# EUROPEAN PATENT APPLICATION

(11) **EP 4 268 830 A1**
(43) Date of publication of application: **01.11.2023**
(21) Application number: 21910927.9
(22) Date of filing: 22.12.2021
(51) Int. Cl.: A61K 31/712, A61K 31/7125, A61K 48/00, A61P 21/00, C12N 15/113

(54) **PHARMACEUTICAL COMPOSITION**

(30) Priority: 22.12.2020 JP 2020213000
(71) Applicant: RIKEN, Wako-shi, Saitama 351-0198 (JP); Hyogo College Of Medicine, Nishinomiya-shi, Hyogo 663-8501 (JP)
(72) Inventor: INOUE, Haruhisa, Wako-shi, Saitama 351-0198 (JP); SUGA, Mika, Wako-shi, Saitama 351-0198 (JP); KONDO, Takayuki, Wako-shi, Saitama 351-0198 (JP); KIMURA, Takashi, Nishinomiya-shi, Hyogo 663-8501 (JP)
(74) Representative: Maiwald GmbH
(86) International application number: PCT/JP2021/047754
(87) International publication number: WO 2022/138787

(57) **Abstract**

The present invention provides novel antisense oligonucleotide pharmaceutical compositions having higher RNA aggregate-removing activity than conventional pharmaceutical compositions. The pharmaceutical composition of the present invention contains an oligonucleotide having any base sequence of a repeated sequence in which cytosine-adenine-guanine trinucleotides are repeated 5 to 13 times, from the 5' end to the 3' end, and the oligonucleotide has a mixmer structure containing at least two types of RNase H inactive nucleotide analogs. The present invention provides the pharmaceutical composition of the present invention used for the treatment of some symptoms of myotonic dystrophy type 1 disease.

## Description

### [Technical Field]

The present invention relates to pharmaceutical compositions, specifically, pharmaceutical compositions containing antisense oligonucleotides. The pharmaceutical composition of the present invention can be used for the treatment of some symptoms of myotonic dystrophy type 1 disease.

### [Background Art]

Myotonic dystrophy type 1 (hereinafter to be referred to as "DM1") is one of the diseases with the highest prevalence among muscular dystrophies. The prevalence of DM1 varies from country to country, and is reported to be 1 in 2,000 to 8,000 people, and 1 in 500 people in some regions (Non Patent Literature 1). DM1 is caused by expansion of the repeated sequences of CTG trinucleotide in the 3' untranslated region (3'UTR) of the myotonic dystrophic protein kinase (hereinafter to be referred to as "DMPK") gene. The DMPK gene encodes a non-receptor serine/threonine kinase protein, and the DMPK protein is ubiquitously expressed throughout the body and strongly expressed in skeletal muscle and cardiac muscle. The specific function of DMPK protein is unknown. DM1 patients have 50-2,000 repeats of CTG repeated sequence in the DMPK gene in most cells, whereas unaffected individuals have only 5-38 CTG repeated sequences (Non Patent Literature 1). The number of CTG sequence repeats roughly correlates with the severity and time of onset of the disease. It has been reported that DMPK RNA extended by CUG repeats forms RNA aggregate (foci) in the cell nuclei of affected individuals. RNA aggregates adsorb and deplete RNA-binding molecules from the nucleus, including CUG-binding protein 1 (CUG-BP1) and muscleblind-like protein 1 (MBNL1), thus impairing many cellular mechanisms such as selective splicing, transcription, translation, and post-translational regulation (Non Patent Literature 2). In addition to DM1, many diseases showing expansion of trinucleotide repeats have been reported, and they are collectively referred to as triplet diseases.

Antisense oligonucleotide (hereinafter to be referred to as "ASO") treatment is one of the promising therapeutic strategies for diseases caused by abnormal RNA in cells and/or nuclei, including mRNA and non-coding RNA, for example, triplet disease (Non Patent Literatures 3 and 4). ASO causes cleavage of target RNA strands by RNase H in the nucleus and cytoplasm, or sterically hinders the association of the target RNA with RNA-binding molecules. ASOs containing various nucleotide analogs have been developed to increase the stability and target binding affinity of single-stranded DNA consisting only of deoxyribonucleotide. The modifications of nucleotides used in ASO include phosphorothioate (PS) modification of the phosphodiester skeleton (hereinafter also referred to as "S"), methylation modification at the 5' position in the pyrimidine ring of the base part of cytidine (hereinafter referred to as "5mC"), substitution of the sugar moiety of any nucleotide with a morpholino ring, substitution of a hydroxyl group at the 2' position of ribose of the sugar moiety of any nucleotide with a fluoro group (hereinafter referred to as "2'-F"), modification with a methoxy group (hereinafter referred to as "2'-OMe"), and modification with an O-methoxyethyl group (hereinafter referred to as "MOE"); and bridge modification of the 2'-position oxygen atom and the 4'-position carbon atom of ribose (for example, a locked nucleic acid (hereinafter referred to as "LNA") in which the oxygen atom at the 2'-position and the carbon atom at the 4'-position of the ribose ring are methylene bridged to fix the conformation of the furanose ring to the C3'-endo type), 2'-O,4'-C-ethylene-bridged nucleic acid in which the 2'-position oxygen atom and the 4'-position carbon atom of ribose are ethylene-bridged (hereinafter referred to as "ENA"), and 2',4'-(S)-constrained ethyl nucleotide analogues (hereinafter referred to as "cEt") in which the oxygen atom at the 2'-position and the carbon atom at the 4'-position of ribose are methyl (methyleneoxy) bridged (4'-CH(CH3)-O-2'). Even though nucleotide analogues may cause harmful side effects when administered into the body, they are advantageous over unmodified deoxyribonucleotides in that they require less dosage, since nucleotide analogues have higher binding affinity to complementary RNA than unmodified deoxyribonucleotides, and oligonucleotides are often more resistant to nucleases.

When a hetero double stranded nucleic acid formed by pairing an oligonucleotide consisting of a specific type of nucleotide analogue with an RNA complementary to the oligonucleotide is degraded by reaction with RNase H under predetermined conditions, the specific type of nucleotide analog is called an RNase H active nucleotide. When a hetero double stranded nucleic acid formed by pairing an oligonucleotide consisting of a specific type of nucleotide analogue with an RNA complementary to the oligonucleotide is not degraded by reaction with RNase H under predetermined conditions, the specific type of nucleotide analog is called an RNase H inactive nucleotide. RNase H active nucleotides contained in the oligonucleotides of the present invention include, but are not limited to, unmodified deoxyribonucleotides and PS-modified deoxyribonucleotide analogs. RNase H inactive nucleotides contained in the oligonucleotides of the present invention include, but are not limited to, morpholino ring-substituted nucleotide analogs, 2'-OMe-nucleotide analogs, MOE-nucleotide analogs, 2'-F nucleotide analogs, LNA, ENA, and cEt.

In the design of ASO, it is known that even ASOs with the same base sequence may have different properties depending on the arrangement of RNase H active nucleotides and RNase H inactive nucleotides. The gapmer structure is a structure in which an oligonucleotide consisting only of RNase H inactive nucleotides (hereinafter to be referred to as "wing region") is connected to the 5' end side and the 3' end side of an oligonucleotide consisting only of RNase H active nucleotides (hereinafter to be referred to as "gap region"). Since the wing region has a high binding affinity to complementary RNA, a stable double strand with the target RNA is formed. Since RNase H active nucleotides are continuous in the gap region, the double strand with the target RNA is degraded by RNase H. Therefore, it is preferable to employ a gapmer structure for ASO that affords efficacy when the double strand with RNA is degraded. Conversely, for ASO that affords efficacy when the double strand with RNA is not degraded, it is preferable to use an oligonucleotide with a structure in which RNase H inactive nucleotides are distributed over the entire length of the ASO and no clear gap region is found. In the present invention, the mixmer structure may be either a structure consisting only of the aforementioned RNase H inactive nucleotides or a structure in which RNase H active nucleotides and RNase H inactive nucleotides are mixed, but the double strand of ASO and the target RNA is not degraded by RNase H since the RNase H inactive nucleotide is arranged in the vicinity of the RNase H active nucleotide. The RNase H inactive nucleotide contained in the mixmer of the present invention may contain oligonucleotides in which all nucleotides have the same modified sugar and all bonds between nucleotides are the same modified bonds, or may contain multiple nucleotide derivatives with different modified sugars and/or modified bonds. The gapmer and mixmer of the present invention respectively refer to oligonucleotides having a gapmer structure and a mixmer structure.

It has been reported that several kinds of MOE/DNA gapmer ASOs are effective for decreasing DMPK RNA with expanded repeated sequence of the CUG sequence in in vitro and in vivo DM1 disease models (Non Patent Literatures 5 and 6). It has been reported that 2'-OMe-modified ASOs (Non Patent Literatures 7 and 8) and morpholino-substituted ASOs (Non Patent Literatures 8 and 9) can also mitigate the action of DMPK RNA with expanded repeated sequence of the CUG sequence, in DM disease models. A part of the aforementioned MOE/DNA gapmer ASOs progressed to clinical trial stage, but failed to deliver ASOs at concentrations that exhibit efficacy in vitro (Non Patent Literature 4). Therefore, it is necessary to develop ASOs that exhibit efficacy at lower concentrations.

In experiments investigating the effect of conventional ASOs for DM1 treatment, fibroblasts, muscle satellite cells, or myoblasts derived from DM1 patients were used in a small part of the experiments, and transgenic mice into which the genomic DNA of the human DMPK gene with expanded CTG repeated sequences derived from DM1 patients was introduced, or myoblasts or myotubes derived from such mice were mainly used. However, the CTG repeated sequences of the human DMPK gene introduced into the aforementioned transgenic mouse has a repeat number of about several hundred, which is insufficient as a model of human DM1 disease with a repeat number of more than a thousand, sometimes several thousands.

Patient-derived induced pluripotent stem cells (hereinafter to be referred to as "iPSC" or "iPS cell") resources are useful for drug discovery and development. Multiple iPSCs generated from primary cultures of fibroblasts, myoblasts, urine-derived cells, or immortalized lymphoblastic cells from patients with myotonic dystrophy have been described (Non Patent Literatures 10 to 18). Nuclear RNA aggregates have been observed as a pathological phenotype in iPSCs derived from patients with myotonic dystrophy, and skeletal muscle cells and cardiomyocytes differentiated from iPSCs derived from patients with myotonic dystrophy (Non Patent Literatures 16 to 18). However, the effects of ASO for DM1 treatment has never been investigated using DM1 patient-derived iPS cells or cells differentiated from the iPS cells. Therefore, it is necessary to establish multiple DM1 patient-derived iPS cells with different numbers of repeats of the CTG sequence and to evaluate ASO for DM1 treatment in disease model cell lines that more faithfully reproduce the pathology of human DM1 disease.

Among the nucleotide analogues described above, ENA is a nucleotide analogue that is highly resistant to nuclease degradation and thermodynamically stable. (Non Patent Literatures 8, 19, and 20). ASOs that have incorporated ENA have high affinity for complementary RNA strands (Non Patent Literatures 8, 19, and 20). The ENA/2'-OMe ribonucleotide analog mixmer ASO, which can induce exon skipping by steric hindrance, is under development as a therapeutic drug for Duchenne muscular dystrophy (DMD) (Non Patent Literatures 20 to 22). ASOs of ENA/deoxyribonucleotide gapmer and ENA/2'-OMe ribonucleotide analog mixmer can reduce RNA aggregates in iPS cells derived from patients with spinocerebellar ataxia type 36 (SCA36) and nerve cells derived from the iPS cells (Non Patent Literature 23). Thus, ASOs that have incorporated ENA are particularly promising as therapeutic drugs with an action mechanism mediated by steric hindrance. It is therefore necessary to develop oligonucleotides that have incorporated ENA as ASOs for DM1 treatment.

Furthermore, DM1 is a multiorgan disease that affects not only skeletal muscle and smooth muscle, but also eyes, heart, endocrine system, and central nervous system. Clinical symptoms range from mild to severe, and although there is some overlap, they are classified into three types of mild, classic, and congenital types. Mild DM1 is characterized by cataract and mild myotonia (prolonged state of muscle contraction), and vital prognosis is normal. Classic DM1 is characterized by muscle weakness/atrophy, myotonia, and cataract, and often accompanied by impaired cardiac conduction. In adults, physical function declines and vital prognosis may be shortened. Congenital DM1 is characterized by hypotonia and marked systemic muscle weakness at birth, often resulting in respiratory failure and premature death. It is often accompanied by intellectual disability. In DM1, muscle weakness/atrophy, cardiac muscle disorder, cardiac conduction disorder, cataract, retinal degeneration, gastrointestinal tract symptoms such as swallowing, constipation, and diarrhea associated with smooth muscle disorder, higher brain dysfunction, hyperinsulinemia, diabetes, testicular atrophy, endocrine disorders such as abnormal secretion of growth hormone, and skin symptoms such as pilomatorixoma and epithelioma have also been reported. Accordingly, treatment of organs other than muscle may be required depending on the clinical symptoms of the patient. For this reason, there is also a need to develop ASO for DM1 treatment targeting not only skeletal muscle but also satellite cells that are progenitor cells of skeletal muscle always present in muscle tissue, tissues other than muscle, for example, ocular tissues (lens cells that cause cataract, retinal cells that cause retinal degeneration, etc.), nerves, skin, endocrine system, and other tissues.

### [Citation List]

### [Non Patent Literature]

[NPL 1]
   Chakraborty, S. et al., Current Protocols in Human Genetics, 91: 9.29.1-9.29.19. (2016)
[NPL 2]
   Pettersson, O.J. et al., Nucleic Acids Research, 43: 2433-2441. (2015)
[NPL 3]
   Crooke, S.T. et al., Cell Metabolism, 27: 714-739. (2018)
[NPL 4]
   Overby, S.J. et al., Drug Discovery Today 23: 2013-2022. (2018)
[NPL 5]
   Wheeler, T.M. et al., Nature, 488: 111-115. (2012)
[NPL 6]
   Jauvin, D. et al., Mol Ther Nucleic Acids, 7: 465-474. (2017)
[NPL 7]
   Mulders, S.A. et al., Proc Natl Acad Sci U S A, 106: 13915-13920. (2009)
[NPL 8]
   Gonzalez-Barriga, A. et al., Mol Ther Nucleic Acids, 2: e81. (2013)
[NPL 9]
   Klein, A.F. et al., J Clin Invest, 129: 4739-4744. (2019)
[NPL 10]
   Du, J. et al., Hum Mol Genet, 22: 5276-5287. (2013)
[NPL 11]
   Xia, G. et al., Cellular reprogramming, 15: 166-177. (2013)
[NPL 12]
   Xia, G. et al., Stem Cells, 33: 1829-1838. (2015)
[NPL 13]
   Gao, Y, Mol Ther, 24: 1378-1387. (2016)
[NPL 14]
   Ueki, J. et al., Sci Rep, 7: 42522. (2017)
[NPL 15]
   Martineau, L. et al., Stem Cell Res, 26: 103-106. (2018)
[NPL 16]
   Mondragon-Gonzalez, R. and Perlirigeiro, R.C.R., Dis Model Mech, 11: dmm034728. (2018)
[NPL 17]
   Dastidar, S. et al., Nucleic Acids Res., 46: 8275-8298. (2018)
[NPL 18]
   Kim, E.Y. et al., JCI Insight, 4: e122686. (2019)
[NPL 19]
   Morita, K. et al., Bioorganic & Medicinal Chemistry, 11: 2211-2226. (2003)
[NPL 20]
   Lee, T. et al., Genes, 8: 67. (2017)
[Non Patent Literature 21]
   Malueka, R.G. et al., Nucleic Acid Therapeutics, 21: 347-353. (2011)
[Non Patent Literature 22]
   Shoji, E. et al., Sci Rep, 5: 12831.(2015)
[Non Patent Literature 23]
   Matsuzono, K. et al., Mol Ther Nucleic Acids, 8: 211-219. (2017)

### [Summary of Invention]

### [Technical Problem]

An object of the present invention is to provide a novel antisense oligonucleotide therapeutic agent for myotonic dystrophy type 1 disease, that has higher RNA aggregate removing activity than conventional agents.

### [Solution to Problem]

The present inventors have conducted intensive studies in an attempt to achieve the above-mentioned objects and found that a disease model system using iPS cells derived from myotonic dystrophy type 1 disease patients is suitable for screening antisense oligonucleotides with high RNA aggregate removing activity, and completed the present invention by using the assay system.

The present invention provides pharmaceutical compositions. The pharmaceutical composition of the present invention contains an oligonucleotide having any base sequence of a repeated sequence in which cytosine-adenine-guanine trinucleotides are repeated 5 to 13 times, from the 5' end to the 3' end, and the oligonucleotide has a mixmer structure containing at least two types of RNase H inactive nucleotide analogs.

In the pharmaceutical composition of the present invention, the aforementioned oligonucleotide may contain
(1) an oligonucleotide consisting of a base sequence of any of SEQ ID NOs: 1 to 9,
(2) an oligonucleotide consisting of a base sequence consisting of adenine-guanine, a base sequence of any of SEQ ID NOs: 1 to 9, and cytosine, from the 5' end to the 3' end, or
(3) an oligonucleotide consisting of a base sequence consisting of guanine, a base sequence of any of SEQ ID NOs: 1 to 9, and cytosine-adenine, from the 5' end to the 3' end.

In the pharmaceutical composition of the present invention, the aforementioned RNase H inactive nucleotide analog may be a nucleotide analog modified at the 2'-position of ribose and/or a modified nucleotide analog bridged between the 2'-position and the 4'-position of ribose.

In the pharmaceutical composition of the present invention, the aforementioned nucleotide analog with a modified oxygen atom at the 2'-position of ribose may be a 2'-OMe-nucleotide analog and/or a MOE-nucleotide analog.

In the pharmaceutical composition of the present invention, the aforementioned modified nucleotide analog bridged between the 2'-position and the 4'-position of ribose may be β-D-oxy-L-LNA, β-D-ENA and/or R type cEt.

In the pharmaceutical composition of the present invention, the aforementioned nucleotide analogs modified at the 2'-position of ribose may all be the same modified sugar.

In the pharmaceutical composition of the present invention, the aforementioned nucleotide analogs modified at the 2'-position of ribose may be at least two different modified sugars.

In the pharmaceutical composition of the present invention, the aforementioned modified nucleotide analogs bridged between the 2'-position and the 4'-position of ribose may all be the same modified sugar.

In the pharmaceutical composition of the present invention, the aforementioned modified nucleotide analogs bridged between the 2'-position and the 4'-position of ribose may be at least two different modified sugars.

In the pharmaceutical composition of the present invention, the aforementioned oligonucleotide may contain β-D-ENA-cytidine, 2'-OMe-adenosine, and 2'-OMe-guanosine.

In the pharmaceutical composition of the present invention, the cytidines in the aforementioned oligonucleotide may all be β-D-ENA-cytidines, adenosines may all be 2'-OMe-adenosines, and guanosines may all be 2'-OMe-guanosines.

In the pharmaceutical composition of the present invention, the aforementioned oligonucleotide may contain at least one type of nucleotide bond selected from the group consisting of phosphorothioate, phosphorodithioate, and boranophosphate.

The pharmaceutical composition of the present invention may contain a pharmaceutically acceptable carrier or diluent.

In the pharmaceutical composition of the present invention, the aforementioned carrier may contain a cellular membrane permeation carrier and/or a carrier for intranuclear delivery.

The pharmaceutical composition of the present invention can be delivered by parenteral administration selected from the group consisting of transdermal administration, intraocular administration, intra-lens administration, gastrointestinal tract intramucosal administration, gastrointestinal tract submucosal administration, subcutaneous administration, intravenous administration, intraarterial administration, intramuscular administration, intraperitoneal administration, intracranial administration, and intrathecal administration.

The pharmaceutical composition of the present invention can be used for treating any of the symptoms of myotonic dystrophy type 1 disease.

The present invention provides a method for treating myotonic dystrophy type 1 disease by administering an oligonucleotide that has any base sequence of a repeated sequence in which cytosine-adenine-guanine trinucleotides are repeated 5 to 13 times, from the 5' end to the 3' end, and has a mixmer structure containing at least two types of RNase H inactive nucleotide analogs, to patients in need of the oligonucleotide.

The present invention provides an assay system of antisense oligonucleotide therapeutic agents for myotonic dystrophy type 1 disease, containing a cell derived from an iPS cell established from a human affected with a myotonic dystrophy type 1 disease. The aforementioned iPS cell-derived cell includes undifferentiated pluripotent stem cells, cells differentiated into at least one cell type, and an aggregate containing the differentiated cells.

### [Brief Description of Drawings]

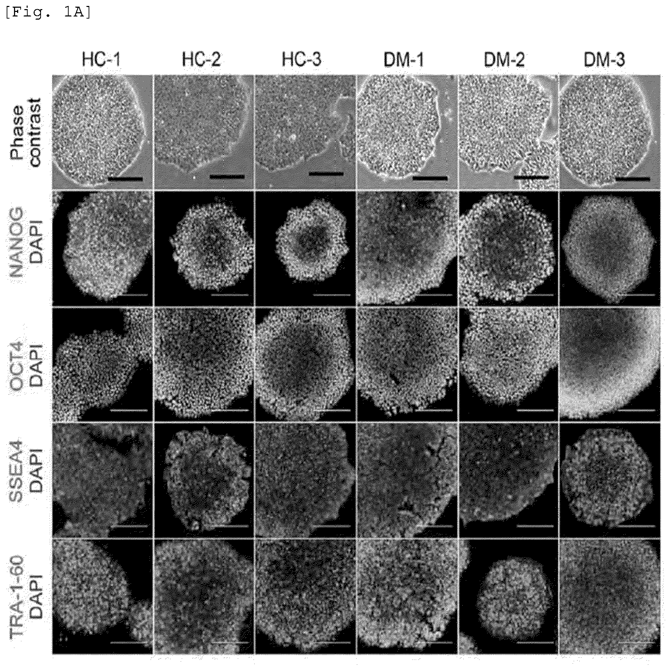

Fig. 1A is a panel of microscope photographs showing the expression of undifferentiated pluripotent stem cell markers in iPS cells (DM-1 to -3) derived from DM1 patient and iPS cells (HC-1 to -3) derived from healthy subject. The top row shows phase contrast microscope photographs of monolayer culture of the undifferentiated cells of respective iPS cells, and the second row shows fluorescence microscope photographs of monolayer culture of the undifferentiated cells of respective iPS cells, that were stained with an antibody against NANOG and then subjected to counterstaining of the cell nuclei with 4',6-diamidino-2-phenylindole (hereinafter to be referred to as "DAPI"). The third row shows fluorescence microscope photographs of monolayer culture of the undifferentiated cells of respective iPS cells, that were stained with an antibody against OCT4 and then subjected to counterstaining of the cell nuclei with DAPI. The fourth row shows fluorescence microscope photographs of monolayer culture of the undifferentiated cells of respective iPS cells, that were stained with an antibody against SSEA4 and then subjected to counterstaining of the cell nuclei with DAPI. The fifth row shows fluorescence microscope photographs of monolayer culture of the undifferentiated cells of respective iPS cells, that were stained with an antibody against TRA-1-60 and then subjected to counterstaining of the cell nuclei with DAPI.

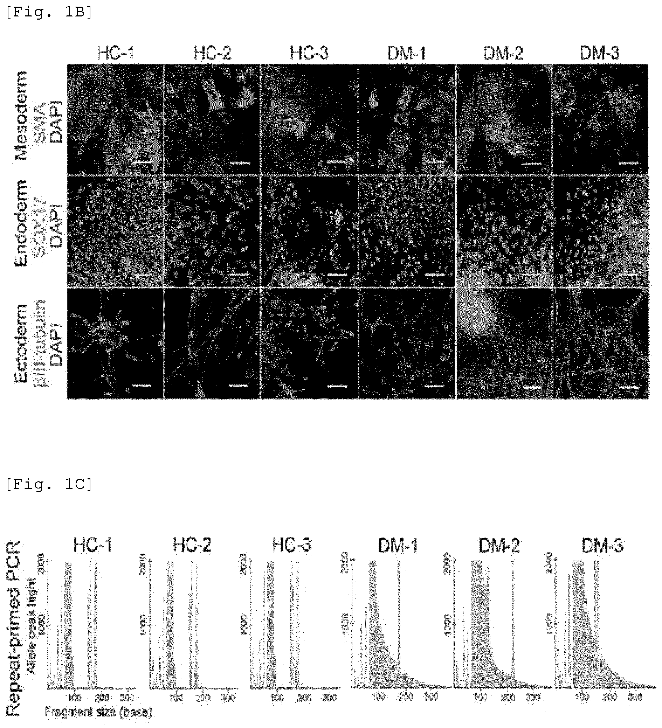

Fig. 1B is a panel of microscope photographs showing the expression of three germ layer markers in cells differentiated from iPS cells (DM-1 to -3) derived from DM1 patient and iPS cells (HC-1 to -3) derived from healthy subject. The first row shows fluorescence microscope photographs of cells differentiated into mesoderm from respective iPS cells, that were stained with an antibody against α-smooth muscle actin (SMA) and then subjected to counterstaining of the cell nuclei with DAPI. The second row shows fluorescence microscope photographs of cells differentiated into endoderm from respective iPS cells, that were stained with an antibody against SOX17 and then subjected to counterstaining of the cell nuclei with DAPI. The third row shows fluorescence microscope photographs of cells differentiated into ectoderm from respective iPS cells, that were stained with an antibody against βIII-tubulin and then subjected to counterstaining of the cell nuclei with DAPI.
Fig. 1C shows capillary electrophoresis of reaction products of triplet repeat prime PCR (hereinafter referred to as "TP-PCR") of iPS cells (DM-1 to -3) derived from DM1 patient and iPS cells (HC-1 to -3) derived from healthy subject.

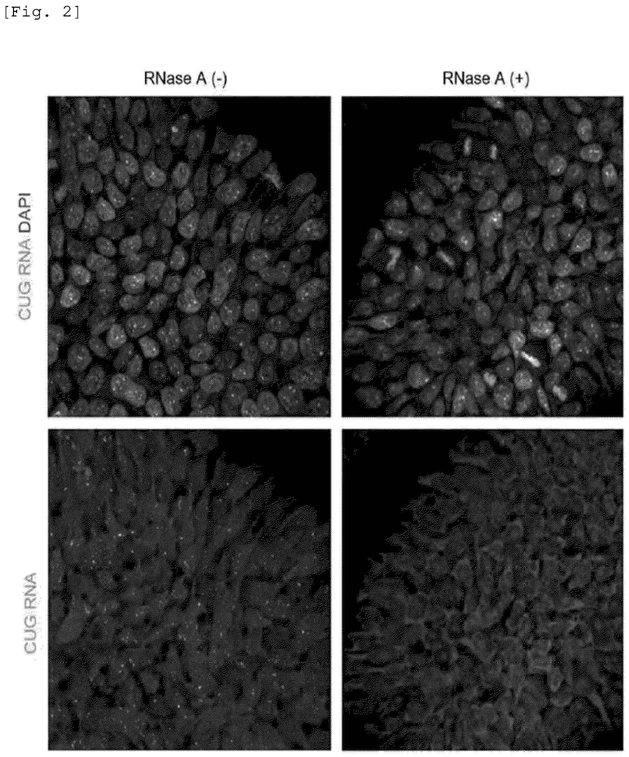

Fig. 2 is a panel of fluorescence microscope photographs showing the results of fluorescence in situ hybridization (hereinafter to be referred to as "FISH") of iPS cells (DM-3) derived from DM1 patient, using Cy3 labeled (CAG)₆-CA DNA/LNA probe. The upper left and the lower left show the results of FISH without RNase A treatment, and the upper right and the lower right show the results of FISH after RNase A treatment. The upper left and the upper right are fluorescence microscope photographs with counterstaining of the cell nuclei with DAPI after FISH, and the lower left and the lower right are fluorescence microscope photographs with FISH alone and without DAPI staining.

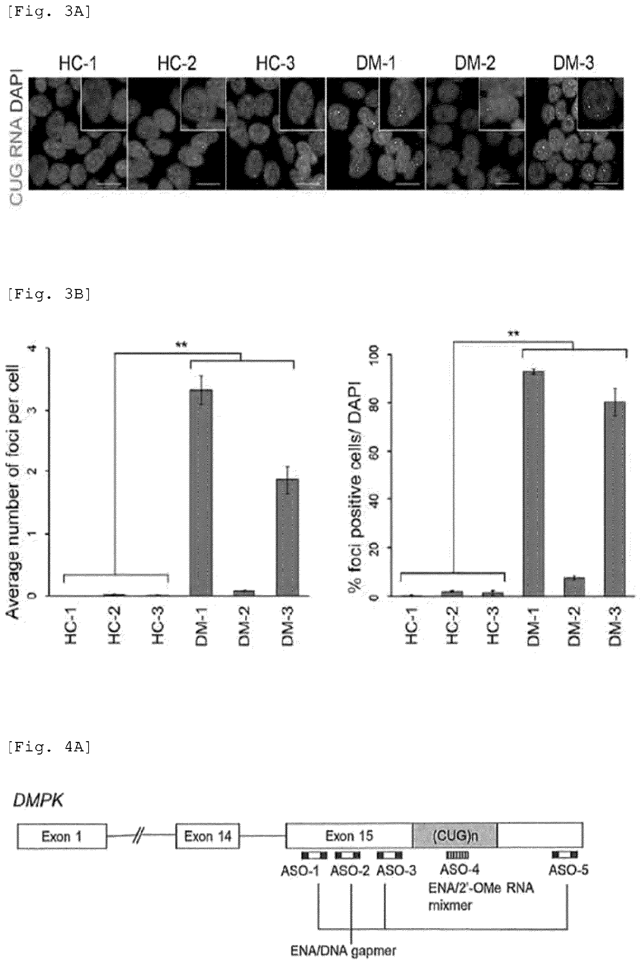

Fig. 3A is a panel of fluorescence microscope photographs showing the results of FISH of respective undifferentiated cells of iPS cells (DM-1 to -3) derived from DM1 patient and iPS cells (HC-1 to -3) derived from healthy subject, using Cy3 labeled (CAG)₆-CA DNA/LNA probe. The upper right corner of each photograph is a high magnification fluorescence microscope photograph of one nucleus.
Fig. 3B, left, is a bar graph showing the average number of RNA aggregates per cell nucleus calculated from the FISH results in Fig. 3A. Fig. 3B, right, is a bar graph showing the percentage of RNA aggregate-positive cell nuclei detected by DAPI staining in total cell nuclei, calculated from the FISH results in Fig. 3A.
Fig. 4A is a schematic diagram showing the relative positional relationship on DMPK-pre mRNA of the complementary sequences of ASOs of the Examples of the present application, and also showing the structural types of ASOs. ASO-1 to -3 are complementary to sequences in the noncoding region of exon 15 on DMPK-mRNA, ASO-4 is complementary to repeated sequence of CUG sequence in the noncoding region of exon 15 on DMPK-mRNA, and ASO-5 is complementary to sequence on the 3' end side of the repeated sequence in the noncoding region of exon 15 on DMPK-mRNA. ASO-4 is a mixmer consisting of ENA and 2'-OMe-nucleotide analog. Other ASO-1 to -3, and 5 are gapmers consisting of ENA in the wing region and unmodified deoxyribonucleotides in the gap region (hereinafter to be referred to as "ENA/DNA gapmer").

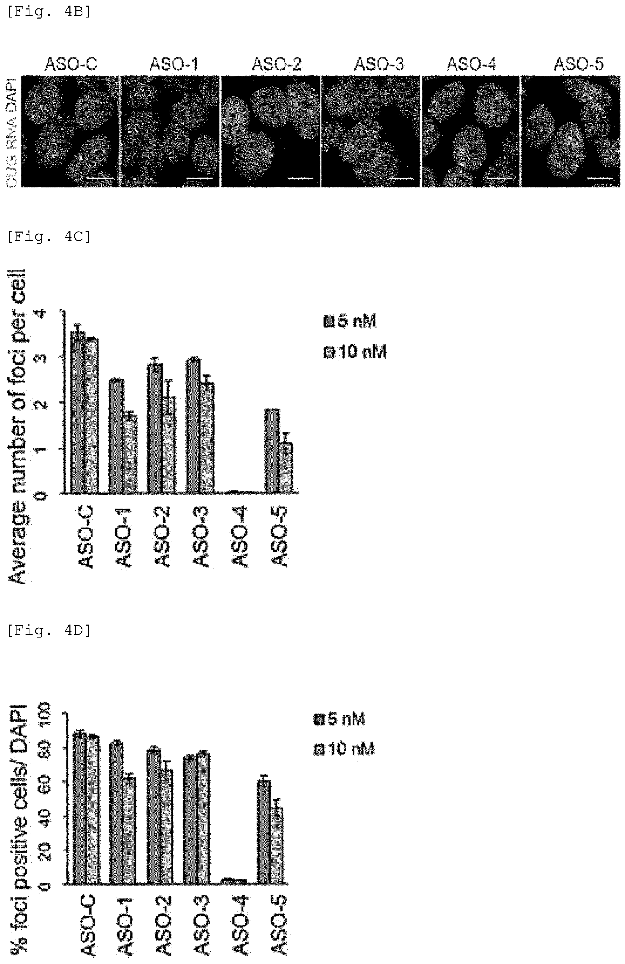

Fig. 4B is a fluorescence microscope photograph of iPS cells (DM-1) derived from DM1 patient and administered with either ASO-C or ASO-1 to -5, followed by FISH using Cy3 labeled (CAG)₆-CA DNA/LNA probe and DAPI counterstaining 48 hr later.
Fig. 4C is a bar graph showing the average number of RNA aggregates per cell nucleus calculated from the FISH results of iPS cells (DM-1) derived from DM1 patient and administered with 5 nM or 10 nM of either ASO-C or ASO-1 to -5.
Fig. 4D is a bar graph showing the percentage of RNA aggregate-positive cell nuclei detected by DAPI staining in total cell nuclei, calculated from the FISH results of iPS cells (DM-1) derived from DM1 patient and administered with 5 nM or 10 nM of either ASO-C or ASO-1 to -5.

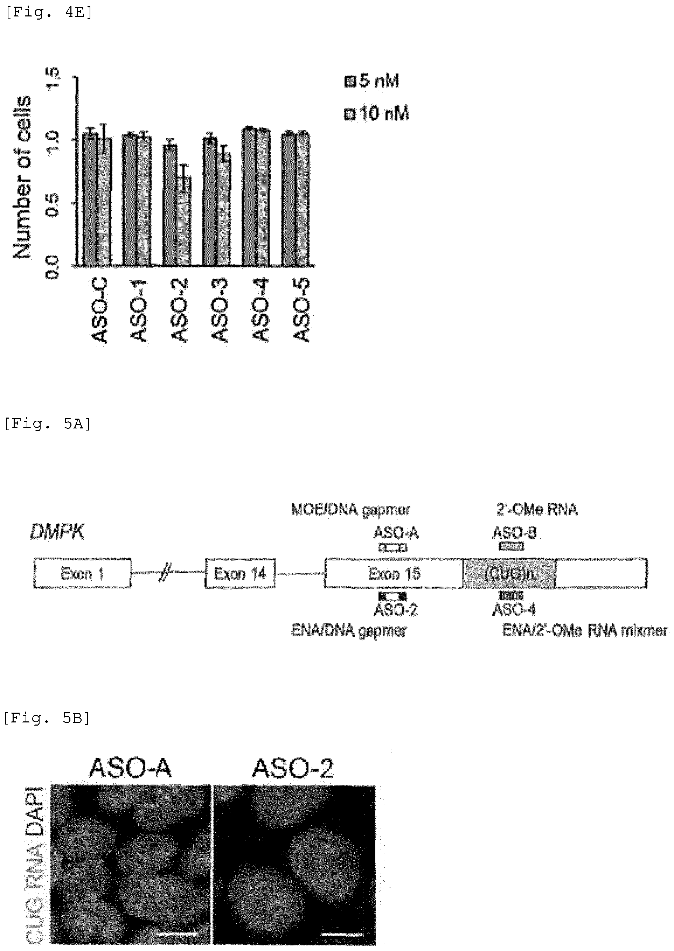

Fig. 4E is a bar graph showing the state of proliferation and survival of iPS cells (DM-1) derived from DM1 patient and administered with 5 nM or 10 nM of either ASO-C or ASO-1 to -5. The vertical axis shows the ratio of the number of cells with the number of ASO-C cells as 1.0.
Fig. 5A is a schematic diagram showing the relative positional relationship on DMPK-pre mRNA of the complementary sequences of ASO-2 and -4, and control ASO-A and -B respectively corresponding thereto of the Examples of the present application, and also showing the structural types of ASOs. ASO-2 and ASO-A are complementary to the same sequence in the coding region of exon 15 on DMPK-mRNA, and ASO-4 and ASO-B are complementary to the same sequence of repeated sequence of CUG sequence in the noncoding region of exon 15 on DMPK-mRNA. ASO-2 is an ENA/DNA gapmer, whereas ASO-A is a gapmer consisting of MOE-nucleotide analog in the wing region and unmodified deoxyribonucleotides in the gap region (hereinafter to be referred to as "MOE/DNA gapmer"). ASO-4 is a mixmer consisting of ENA and 2'-OMe-nucleotide analog, and ASO-B consists of 2'-OMe-nucleotide analogues at all nucleotides in which all bonds between nucleotides are phosphorothioate.
Fig. 5B is a fluorescence microscope photograph of iPS cells (DM-1) derived from DM1 patient and administered with ASO-2 or ASO-A, followed by FISH using Cy3 labeled (CAG)₆-CA DNA/LNA probe and DAPI counterstaining.

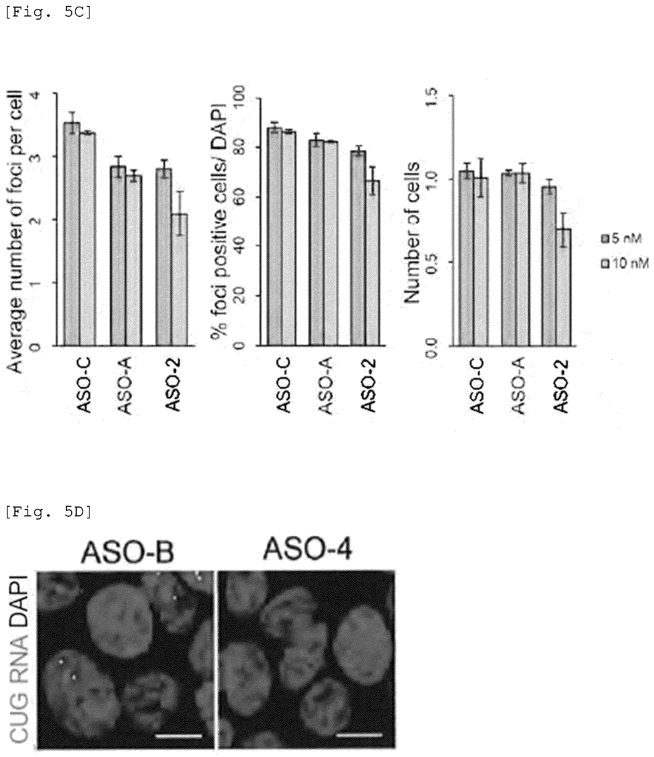

Fig. 5C, left, is a bar graph showing the average number of RNA aggregates per cell nucleus calculated from the FISH results of iPS cells (DM-1) derived from DM1 patient and administered with 5 nM or 10 nM of ASO-2, ASO-A, or ASO-C. Fig. 5C, middle, is a bar graph showing the percentage of RNA aggregate-positive cell nuclei detected by DAPI staining in total cell nuclei, calculated from the FISH results of iPS cells (DM-1) derived from DM1 patient and administered with 5 nM or 10 nM of ASO-2, ASO-A, or ASO-C. Fig. 5C, right, is a bar graph showing the state of proliferation and survival of iPS cells (DM-1) derived from DM1 patient and administered with 5 nM or 10 nM of ASO-2, ASO-A, or ASO-C. The vertical axis shows the ratio of the number of cells with the number of ASO-C cells as 1.0.
Fig. 5D is a fluorescence microscope photograph of iPS cells (DM-1) derived from DM1 patient and administered with ASO-4 or ASO-B, followed by FISH using Cy3 labeled (CAG)₆-CA DNA/LNA probe and DAPI counterstaining.

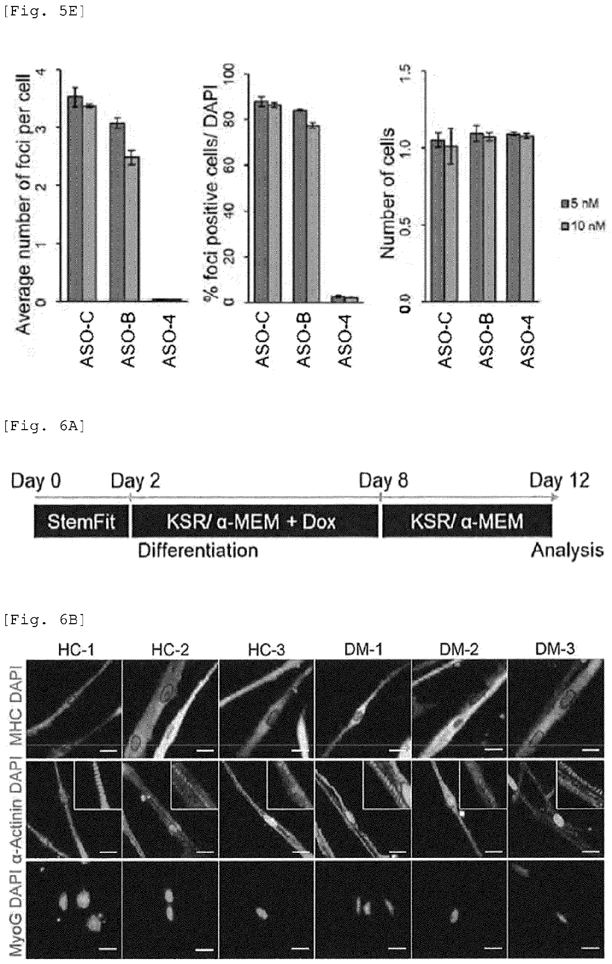

Fig. 5E, left, is a bar graph showing the average number of RNA aggregates per cell nucleus calculated from the FISH results of iPS cells (DM-1) derived from DM1 patient and administered with 5 nM or 10 nM of ASO-4, ASO-B, or ASO-C. Fig. 5E, middle, is a bar graph showing the percentage of RNA aggregate-positive-cell nuclei in total cell nuclei, calculated from the FISH results of iPS cells (DM-1) derived from DM1 patient and administered with 5 nM or 10 nM of ASO-4, ASO-B, or ASO-C. Fig. 5E, right, is a bar graph showing the state of proliferation and survival of iPS cells (DM-1) derived from DM1 patient and administered with 5 nM or 10 nM of ASO-4, ASO-B, or ASO-C. The vertical axis shows the ratio of the number of cells with the number of ASO-C cells as 1.0.
Fig. 6A is a conceptual diagram showing procedures for an experimental system for differentiation of undifferentiated cells of iPS cells (DM-1 to -3) derived from DM1 patient and iPS cells (HC-1 to -3) derived from healthy subject into myocytes, using a tetracycline-inducible MyoD expression system. "StemFit" shows the period of culture in the medium for undifferentiated iPS cells, "KSR/α-MEM" shows the period of culture in a medium for differentiated myocytes, and "KSR/α-MEM+Dox" shows the period during which cells were cultured in a medium for myocytes supplemented with doxycycline as a differentiation-inducing factor. Undifferentiated iPS cells were transfected on day 0 with a tetracycline-inducible MyoD expression system, the medium was changed to a myocyte medium supplemented with a differentiation-inducing factor on day 2 to initiate differentiation induction, the differentiation induction was terminated on day 8 by changing to a myocyte medium containing no differentiation-inducing factor, and analysis was performed on day 12.
Fig. 6B is a panel of fluorescence microscope photographs showing the expression of muscle differentiation marker in cells induced to differentiate from the undifferentiated cells of the cells of HC-1 to -3 and DM-1 to -3 according to the procedures shown in Fig. 6A. The upper panel shows fluorescence microscope photographs of cells stained with an antibody against myosin heavy chain (MHC) and then subjected to counterstaining of the cell nuclei with DAPI. The middle panel shows fluorescence microscope photographs of cells stained with an antibody against α-actinin and then subjected to counterstaining of the cell nuclei with DAPI. The lower panel shows fluorescence microscope photographs of cells stained with an antibody against myogenin (MyoG) and then subjected to counterstaining of the cell nuclei with DAPI.

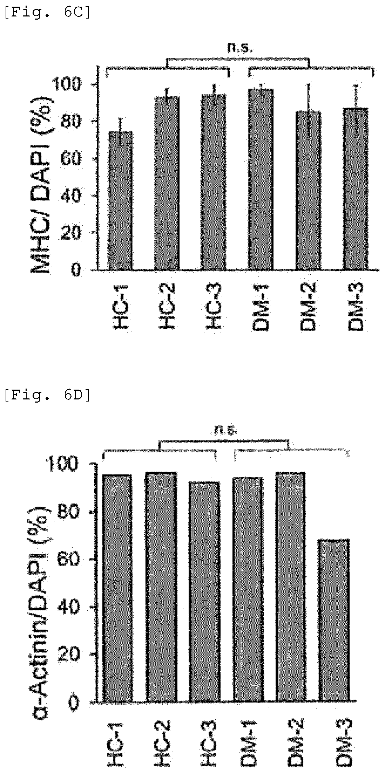

Fig. 6C is a bar graph showing the ratio of MHC-expressing cells in the myocytes differentiated from the cells of HC-1 to -3 and DM-1 to -3 according to the procedures shown in Fig. 6A. The vertical axis shows the percentage of cell nuclei (MHC/DAPI) that express muscle differentiation marker MHC among the DAPI-staining positive cell nuclei induced to differentiate from undifferentiated cells. The "n.s." shows that no significant difference was found between HC-1 to -3 cells and DM-1 to -3 cells.
Fig. 6D is a bar graph showing the ratio of α-actinin-expressing cells in the myocytes differentiated from the cells of HC-1 to -3 and DM-1 to -3 according to the procedures shown in Fig. 6A. The vertical axis shows the percentage of cell nuclei (α-actinin/DAPI) that express muscle differentiation marker α-actinin among the DAPI-staining positive cell nuclei induced to differentiate from undifferentiated cells. The "n.s." shows that no significant difference was found between HC-1 to -3 cells and DM-1 to -3 cells.

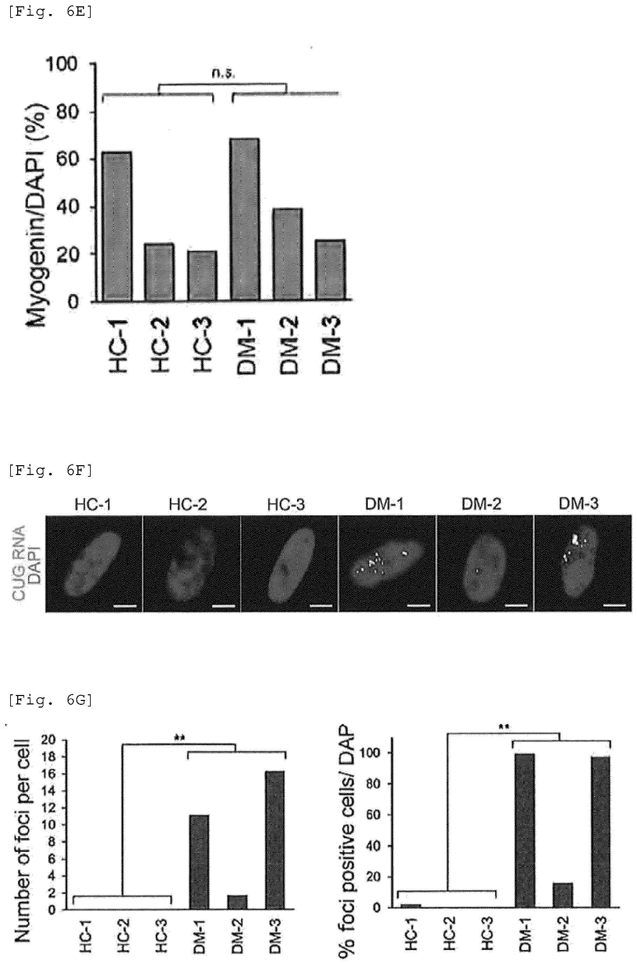

Fig. 6E is a bar graph showing the ratio of myogenin-expressing cells in the myocytes differentiated from the- dells of HC-1 to -3 and DM-1 to -3 according to the procedures shown in Fig. 6A. The vertical axis shows the percentage of cell nuclei (myogenin/DAPI) that express muscle differentiation marker myogenin among the DAPI-staining positive cell nuclei induced to differentiate from undifferentiated cells. The "n.s." shows that no significant difference was found between HC-1 to -3 cells and DM-1 to -3 cells.
Fig. 6F is a panel of fluorescence microscope photographs showing the results of FISH using Cy3 labeled (CAG)₆-CA DNA/LNA probe and DAPI counterstaining of the myocytes differentiated from the cells of HC-1 to -3 and DM-1 to -3 according to the procedures shown in Fig. 6A.
Fig. 6G, left, is a bar graph showing the average number of RNA aggregates per cell nucleus calculated from the FISH results of respective myocytes differentiated from the cells of HC-1 to -3 and DM-1 to -3 according to the procedures shown in Fig. 6A. Fig. 6G, right, is a bar graph showing the percentage of RNA aggregate-positive cell nuclei detected by DAPI staining in total cell nuclei, calculated from the FISH results of respective myocytes differentiated from the cells of HC-1 to -3 and DM-1 to -3 according to the procedures shown in Fig. 6A.

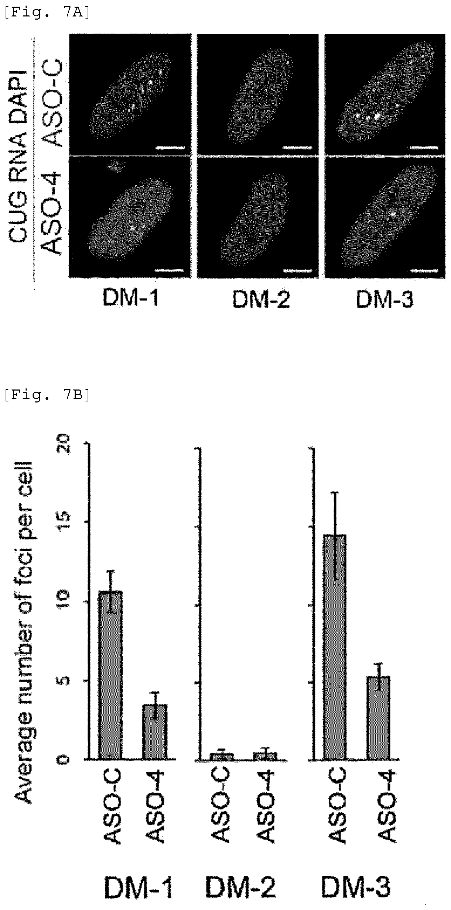

Fig. 7A is a panel of fluorescence microscope photographs showing FISH using Cy3 labeled (CAG)₆-CA DNA/LNA probe and DAPI counterstaining after administration of 10 nM of ASO-C or ASO-4 to myocytes derived from iPS cells of DM1 patient (DM-1 to -3).
Fig. 7B is a bar graph showing the average number of RNA aggregates per cell nucleus calculated from the FISH results of myocytes derived from DM-1 to -3 cells administered with 10 nM of ASO-C or ASO-4.

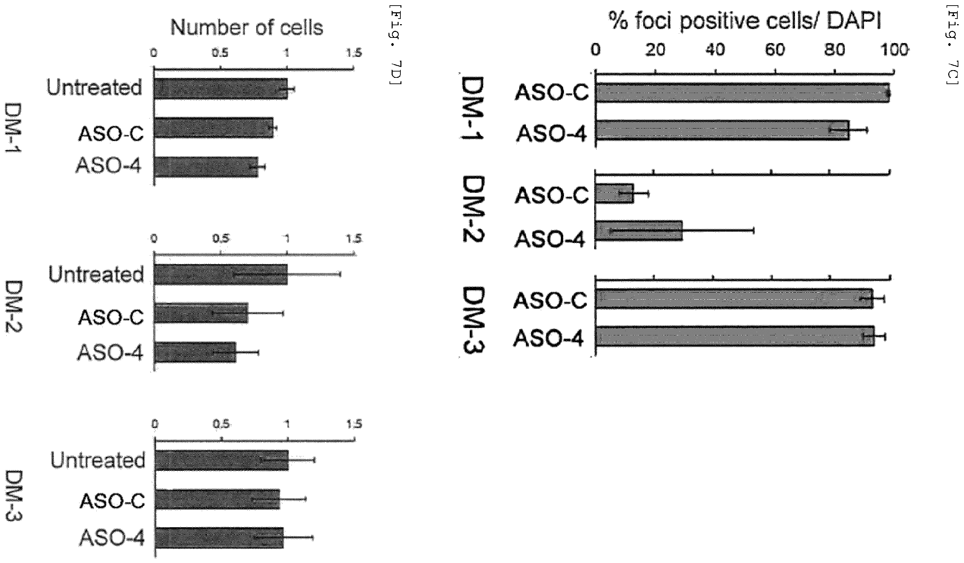

Fig. 7C is a bar graph showing the percentage of RNA aggregate-positive cell nuclei detected by DAPI staining in total cell nuclei, calculated from the FISH results of myocytes derived from DM-1 to -3 cells administered with 10 nM of ASO-C or ASO-4.
Fig. 7D is a bar graph showing-the state of proliferation and survival of myocytes derived from DM-1 to -3 cells administered with 10 nM of ASO-C or ASO-4. In any bar graph, the vertical axis shows the ratio of the number of cells with the number of cells of culture without administration of ASO (Untreated) as 1.0.

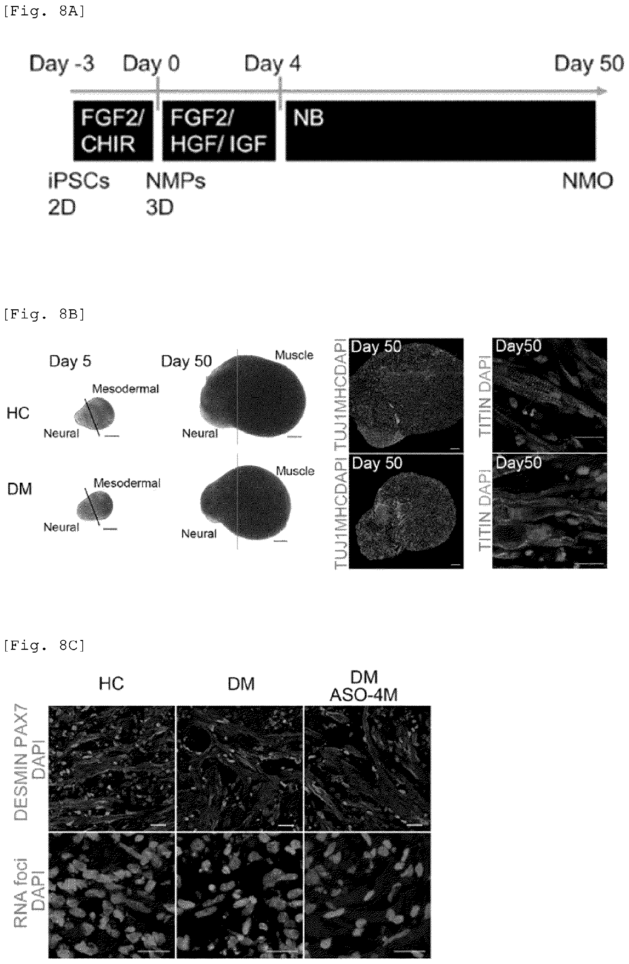

Fig. 8A is a schematic view of the protocol for generating neuromuscular organoids from iPS cells.
Fig. 8B, left, shows phase contrast microscope photograph on day 5 and bright field microscope photograph on day 50 (scale bar is 200 um) of neuromuscular organoid derived from iPS cells of healthy subject (HC, top) and neuromuscular organoid derived from iPS cells of DM1 patient (DM, bottom). The right is a panel of synthetic image of multicolor fluorescence microscope photograph of neuromuscular organoid section on day 50 (scale bar is 20 µm). In the left side of the panel, neuromuscular organoid was stained with anti-myosin heavy chain (MHC) antibody, TUJ1 antibody (anti-β tubulin III antibody), and DAPI. It is divided into an area with many skeletal muscle cells (muscle compartment) and a region with many nerve cells (neural compartment). In the right side of the panel, neuromuscular organoid was stained with anti-TITIN antibody and DAPI. Skeletal muscle cell striations were stained with anti-TITIN antibody. In both cases, the upper row shows neuromuscular organoids (HC) derived from iPS cells of healthy subject, and the lower row shows neuromuscular organoids (DM) derived from iPS cells of DM1 patient.
Fig. 8C, the upper panel shows synthetic images of fluorescence microscope photographs of muscular compartment of neuromuscular organoid multicolor stained with anti-desmin antibody, anti-PAX7 antibody, and DAPI. The lower panel shows synthetic images of fluorescence microscope photographs of muscular compartment of neuromuscular organoid that underwent FISH using Cy3 labeled (CAG)₆-CA DNA/LNA probe and DAPI counterstaining. In both the upper panel and the lower panel, the left is neuromuscular organoid derived from iPS cells of healthy subject (HC) and the right is neuromuscular organoid derived from iPS cells of DM1 patient and treated with ASO-4M (same as ASO-4) (DM ASO-4M). The scale bar is 20 um.

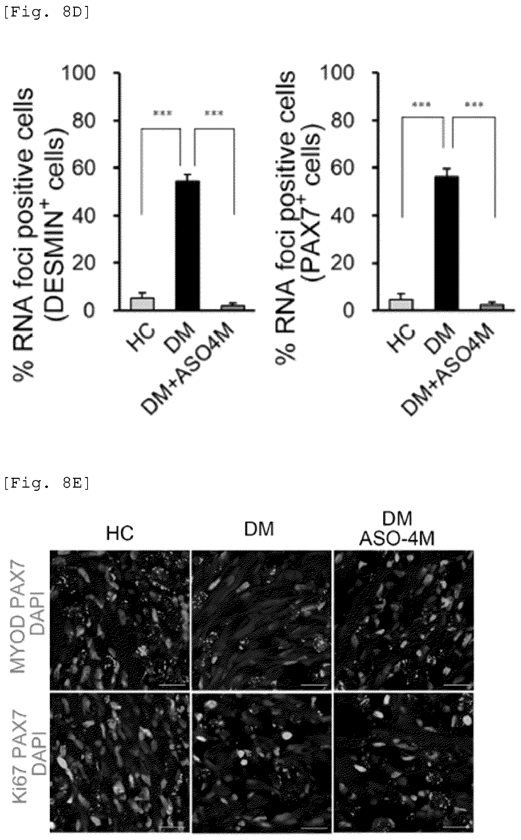

Fig. 8D is a bar graph showing the percentage of cells with RNA aggregates of cells expressing desmin and PAX7. The numerical values are mean±standard deviation (SD) obtained from 5 organoids. "***" indicates significant difference between HC and DM and between DM and DM+ASO4M (independent two-tailed t-test, p<0.001).
Fig. 8E, the upper panel shows synthetic images of fluorescence microscope photographs of muscular compartment of neuromuscular organoid multicolor stained with anti-MYOD antibody, anti-PAX7 antibody, and DAPI. The lower panel shows synthetic images of fluorescence microscope photographs of muscular compartment of neuromuscular organoid multicolor stained with anti-Ki67 antibody, anti-PAX7 antibody, and DAPI. In both the upper panel and the lower panel, the left is neuromuscular organoid derived from iPS cells of healthy subject (HC), the middle is neuromuscular organoid derived from iPS cells of DM1 patient, and the right is neuromuscular organoid derived from iPS cells of DM1 patient and treated with ASO-4M (same as ASO-4) (DM ASO-4M) .

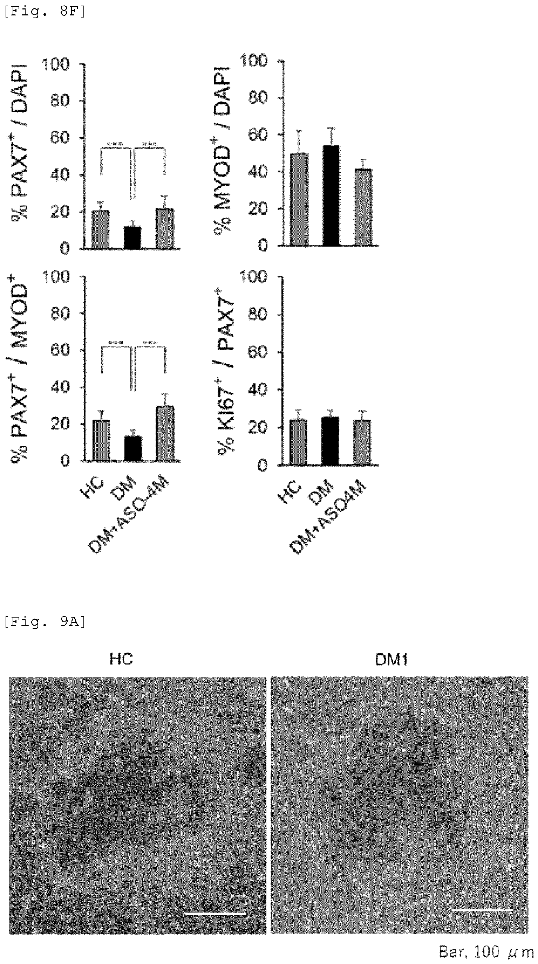

Fig. 8F, upper left, is a bar graph showing the percentage of cells expressing PAX7 among all cells. The upper right is a bar graph showing the percentage of cells expressing MYOD among all cells. The lower left is a bar graph showing the percentage of cells expressing PAX7 among cells expressing MYOD. The lower right is a bar graph showing the percentage of cells expressing Ki67 among cells expressing PAX7. The numerical values are mean±standard deviation (SD) obtained from 5 organoids. "***" indicates significant difference between HC and DM and between DM and DM+ASO4M (independent two-tailed t-test, p<0.001).
Fig. 9A, left, is a phase contrast microscope photograph (HC) of lens epithelial cells and lens fiber cells differentiated from iPS cells of healthy subject. The right is a phase contrast microscope photograph (DM1) of lens epithelial cells and lens fiber cells differentiated from iPS cells of DM1 patient. The scale bar is 100 um.

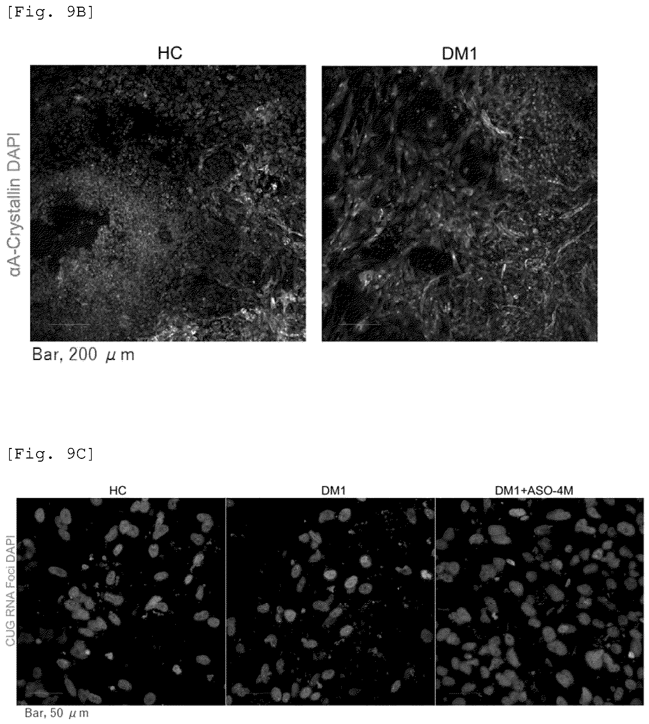

Fig. 9B, left, is a fluorescence microscope photograph (HC) of lens epithelial cells and lens fiber cells differentiated from iPS cells of healthy subject and stained with anti-αA crystallin antibody. The right is a fluorescence microscope photograph (DM1) of lens epithelial cells and lens fiber cells differentiated from iPS cells of DM1 patient and stained with anti-αA crystallin antibody. The scale bar is 200 um.
Fig. 9C, left, is a fluorescence microscope photograph (HC) of lens epithelial cells and lens fiber cells differentiated from iPS cells of healthy subject that underwent FISH using Cy3 labeled (CAG)₆-CA DNA/LNA probe and DAPI counterstaining. The middle is a fluorescence microscope photograph (DM1) of lens epithelial cells and lens fiber cells differentiated from iPS cells of DM1 patient that underwent FISH using Cy3 labeled (CAG)₆-CA DNA/LNA probe and DAPI counterstaining. The right is a fluorescence microscope photograph (DM1+ASO-4M) of lens epithelial cells and lens fiber cells differentiated from iPS cells of DM1 patient and treated with ASO-4M (same as ASO-4) that underwent FISH using Cy3 labeled (CAG)₆-CA DNA/LNA probe and DAPI counterstaining.

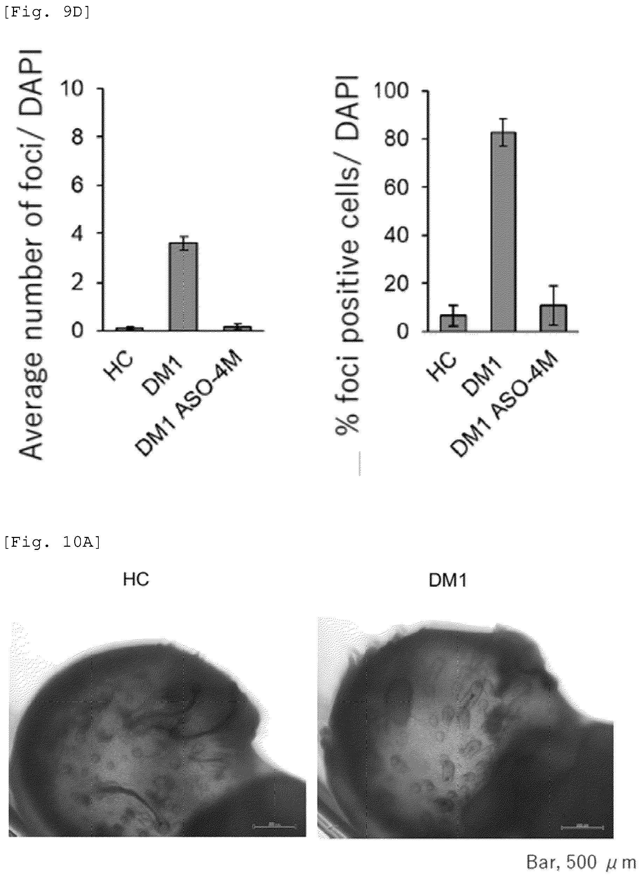

Fig. 9D, left, is a bar graph showing the mean±standard deviation of RNA aggregates per cell nucleus calculated from the FISH results of HC, DM1, and DM1+ASO-4M in Fig. 9C. The right is a bar graph showing the mean±standard deviation of the percentage of RNA aggregates in all cells, calculated from the FISH results of each of HC, DM1, and DM1+ASO-4M in Fig. 9C.
Fig. 10A, left, is a bright field microscope photograph (HC) of skin organoid derived from iPS cells of healthy subject after 4 months of culture under differentiation conditions. The right is a bright field microscope photograph (DM1) of skin organoid derived from iPS cells of DM1 patient. The scale bar is 500 um.

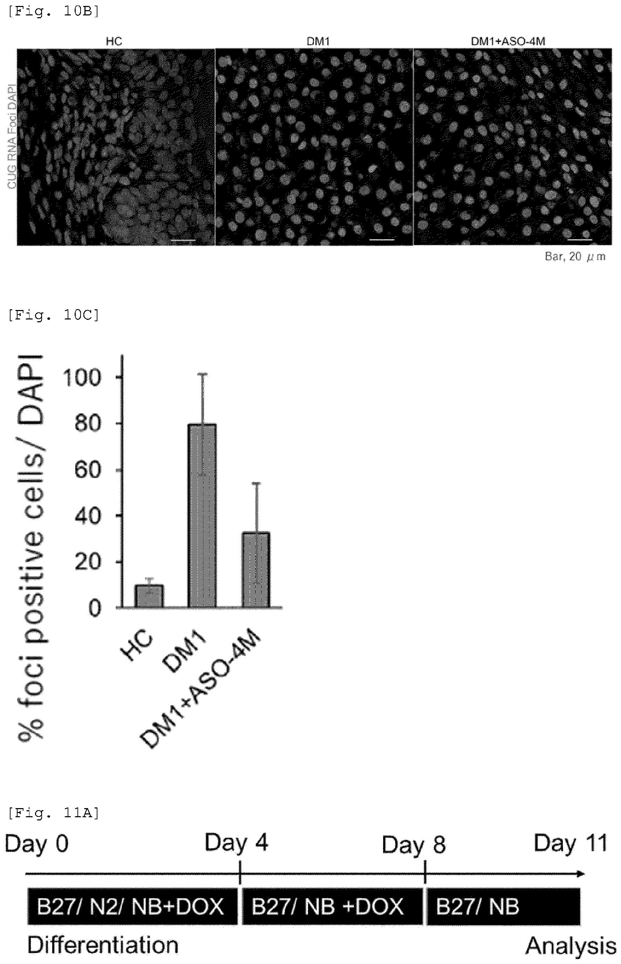

Fig. 10B, left, is a fluorescence microscope photograph (HC) of skin organoid derived from iPS cells of healthy subject that underwent FISH using Cy3 labeled (CAG)₆-CA DNA/LNA probe and DAPI counterstaining. The middle is a fluorescence microscope photograph (DM1) of skin organoid derived from iPS cells of DM1 patient that underwent FISH using Cy3 labeled (CAG)₆-CA DNA/LNA probe and DAPI counterstaining. The right is a fluorescence microscope photograph (DM1+ASO-4M) of skin organoid derived from iPS cells of DM1 patient and treated with ASO-4M (same as ASO-4) that underwent FISH using Cy3 labeled (CAG)₆-CA DNA/LNA probe and DAPI counterstaining. The scale bar is 20 µm.
Fig. 10C is a bar graph showing the mean±standard deviation of the percentage of cells in which RNA aggregates are detected in all cells, calculated from the FISH results of each of HC, DM1, and DM1+ASO-4M in Fig. 10B.
Fig. 11A is a conceptual diagram showing procedures for an experimental system for differentiation of undifferentiated cells of iPS cells (DM-1 to -3) derived from DM1 patient and iPS cells (HC-1 to -3) derived from healthy subject into nerve cells, using a tetracycline-inducible NGN2 expression system. "B27/N2/NB+DOX" and "B27/NB+DOX" show the periods of culture in a medium for nerve cell induction supplemented with doxycycline as a differentiation-inducing factor, and "B27/NB" shows the periods of culture in a medium for differentiated nerve cells. Tetracycline-inducible NGN2expression system was introduced into undifferentiated iPS cells by transfection, the medium was changed to a medium for nerve cell induction, differentiation induction was initiated on day 0, the differentiation induction was terminated on day 8 by changing to a medium for nerve cells without containing a differentiation-inducing factor, and analysis was performed on day 11.

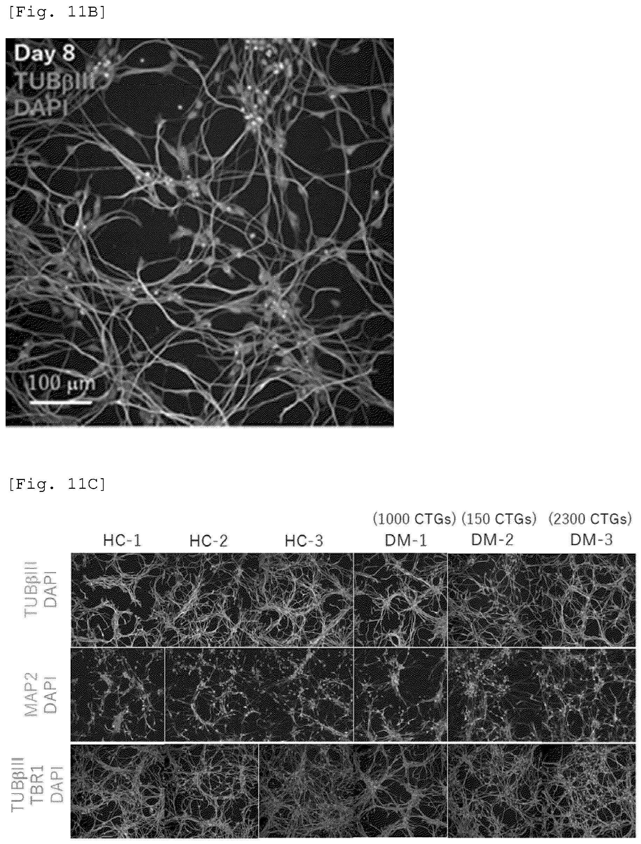

Fig. 11B is a fluorescence microscope photograph of neuron differentiated from iPS cells derived from DM1 patient that underwent staining with an anti-tubulin βIII antibody and DAPI counterstaining. The scale bar is 100 µm.
Fig. 11C shows synthetic images of fluorescence microscope photographs of neurons differentiated from iPS cells (HC-1 to -3) derived from healthy subject and iPS cells (DM-1 to -3) derived from DM1 patient that underwent staining with anti-tubulin βIII antibody (upper row), anti-MAP2 antibody (middle row), or anti-tubulin βIII antibody and anti-Tbr1 antibody, and DAPI counterstaining.

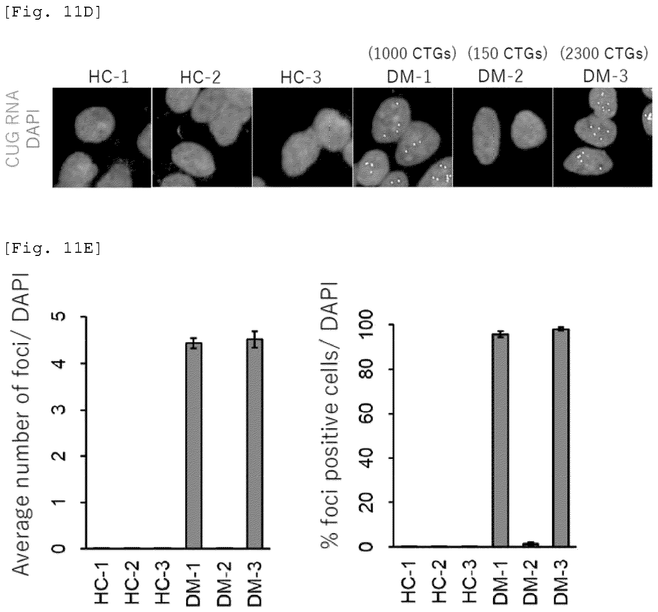

Fig. 11D shows fluorescence microscope photographs (HC) of neurons differentiated from iPS cells (HC-1 to -3) derived from healthy subject and iPS cells (DM-1 to -3) derived from DM1 patient that underwent FISH using Cy3 labeled (CAG)₆-CA DNA/LNA probe and DAPI counterstaining.
Fig. 11E, left, is a bar graph showing the mean±standard deviation of RNA aggregates per cell nucleus calculated from the FISH results of HC-1 to -3 and DM-1 to -3 in Fig. 11C. The right is a bar graph showing the mean±standard deviation of the percentage of RNA aggregates in all cells, calculated from the FISH results of each of HC-1 to -3 and DM-1 to -3 in Fig. 11C.

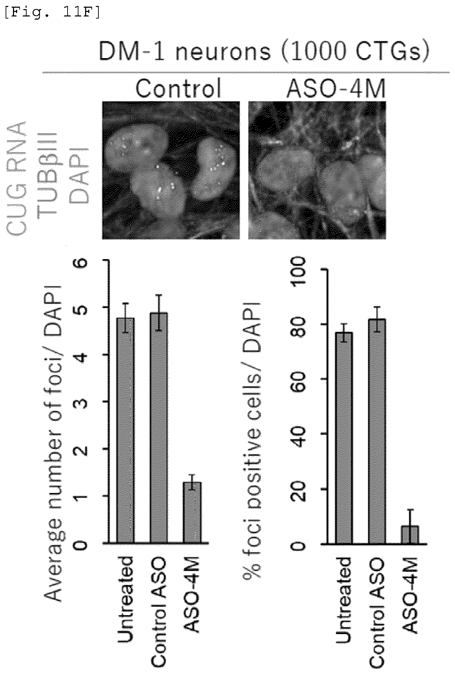

Fig. 11F, upper left, shows a synthetic image (Control) of fluorescence microscope photograph of neurons derived from iPS cells of DM1 patient and treated with Control ASO that underwent FISH using Cy3 labeled (CAG)₆-CA DNA/LNA probe, staining with anti-tubulin βIII antibody, and DAPI counterstaining. The upper right shows a synthetic image (ASO-4M) of fluorescence microscope photograph of neurons derived from iPS cells of DM1 patient and treated with ASO-4M (same as ASO-4) that underwent FISH using Cy3 labeled (CAG)₆-CA DNA/LNA probe, staining with anti-tubulin βIII antibody, and DAPI counterstaining. The lower left is a bar graph showing the mean±standard deviation of RNA aggregates per cell nucleus of neurons derived from iPS cells of DM1 patient and treated with ASO-4M or Control ASO, or without ASO treatment, calculated from the FISH results of Fig. 11F, upper (ASO-4M, Control ASO, or Untreated, respectively). The lower right is a bar graph showing the mean±standard deviation of the percentage of RNA aggregates in all cells of neurons derived from iPS cells of DM1 patient and treated with ASO-4M or Control ASO, or without ASO treatment, calculated from the FISH results of Fig. 11F, upper (ASO-4M, Control ASO, or Untreated, respectively).

### [Description of Embodiments]

One embodiment of the present invention is a 1pharmaceutical composition. The pharmaceutical composition-of the present invention contains an oligonucleotide having any base sequence of a repeated sequence in which cytosine-adenine-guanine trinucleotides are repeated 5 to 13 times, from the 5' end to the 3' end, and the oligonucleotide has a mixmer structure containing at least two types of RNase H inactive nucleotide analogs and/or at least two types of bonds between nucleotides.

The base sequences of the oligonucleotides explained in the present specification are shown in SEQ ID NO: 1 to 23 of the Sequence Listing attached to the present specification. Among those sequences, the correspondence between base sequence of a repeated sequence in which cytosine-adenine-guanine trinucleotides are repeated 5 to 13 times, from the 5' end to the 3' end and SEQ ID NO: in the Sequence Listing attached to the present specification is shown in Table 1.

**[Table 1]**

| SEQ ID NO: | sequence |
|---|---|
| SEQ ID NO: 1 | (CAG)₅ |
| SEQ ID NO: 2 | (CAG)₆ |
| SEQ ID NO: 3 | (CAG)₇ |
| SEQ ID NO: 4 | (CAG)₈ |
| SEQ ID NO: 5 | (CAG)₉ |
| SEQ ID NO: 6 | (CAG)₁₀ |
| SEQ ID NO: 7 | (CAG)₁₁ |
| SEQ ID NO: 8 | (CAG)₁₂ |
| SEQ ID NO: 9 | (CAG)₁₃ |

In Table 1, "(CAG)ₙ" means an oligonucleotide with a trinucleotide repeat number of n in which cytidine, adenosine, and guanosine are linked in order from the 5' end to the 3' end. As used herein, n is any integer from 5 to 13.

The antisense oligonucleotides administered to the iPS cells in the Examples of the present specification are ASO-1 to -5, and the ASOs used as the control are ASO-A to -C. The base sequences of ASO-1 to -3, -4 and -5 are listed in SEQ ID NOs: 11 to 13, 3, and 14, respectively, in the Sequence Listing attached hereto. The base sequences and structural characteristics thereof are shown in Table 2.

**Table 2]**

| | nucleotide sequence and modification | type | structure | **SEQ** ID NO: |
|---|---|---|---|---|
| ASO-C | 5'-_{E}C_{E}C_{E}T_{E}A_{E}TdAdGdGdAdCdTdAdTdCdC_{E}A_{E}G_{E}G_{E}A_{E}A-3' | RNase H active | ENA/DNA Gapmer | SEQ ID NO: 10 |
| ASO-1 | 5'-_{E}G_{E}G_{E}A_{E}G_{E}TdCdGdAdAdGdAdCdAdGdTdT_{E}C_{E}T_{E}A_{E}G_{E}G-3' | RNase H active | ENA/DNA Gapmer | SEQ ID NO: 11 |
| ASO-2 | 5'-_{E}C_{E}G_{E}G_{E}A_{E}GdCdGdGdTdTdGdTdGdAdA_{E}C_{E}T_{E}G_{E}G_{E}C-3' | RNase H active | ENA/DNA Gapmer | SEQ ID NO: 12 |
| ASO-3 | 5'-_{E}G_{E}G_{E}T_{E}A_{E}CdAdCdAdGdGdAdCdTdGdGdA_{E}G_{E}CT_{E}G_{E}G-3' | RNase H active | ENA/DNA Gapmer | SEQ ID NO: 13 |
| ASO-4 | 5'-_{E}CₘAₘG_{E}CₘAₘG_{E}CₘAₘG_{E}CₘAₘG_{E}CmAₘG_{E}CₘAₘG_{E}CₘAₘG-3' | blocker | ENA/2'-CMe RNA Mixmer | **SEQ** ID NO: 3 |
| ASO-5 | 5'-_{E}A_{E}C_{E}AdAdTdAdAdAdTdAdCdCdG_{E}A_{E}G_{E}G-3' | RNase H active | ENA/DNA Gapmer | SEQ ID NO: 14 |

In the structure of each ASO in Table 2, "A", "G", and "C" are adenosine, guanosine, and cytidine, respectively, "dA", "dG", "dC", and "dT" are deoxyadenosine, deoxyguanosine, deoxycytidine, and thymidine, respectively, adjacent nucleotides are linked by a phosphodiester bond, nucleotide to the right of the subscript "E" is β-D-ENA-nucleic acid (hereinafter to be referred to as "ENA"), and nucleotide to the right of the subscript "m" is 2'-O-methyl (hereinafter to be referred to as "2'-OMe") nucleotide analog.

The nucleotide analogs that can be used in the ASO of the present invention are described in detail below.

Among the aforementioned ASOs, ASO-C, ASO-1 to -3, and ASO-5 have a gapmer (hereinafter to be referred to as "ENA/DNA gapmer") structure in which the nucleoside constituting the wing region is ENA and the nucleoside constituting the gap segment is DNA. In ASO-1 to -3 and ASO-5, all nucleotides of the gapmers are paired with the RNA of the DMPK gene of myotonic dystrophy type 1 patients, and the nucleotides constituting the gap segments are DNA. Thus, when the aforementioned gapmer is paired with the RNA of the DMPK gene, the gap segment part becomes a hetero double strand of DNA and RNA and is degraded by RNase H. On the other hand, ASO-4 has a mixmer structure, in which β-D-ENA-modified cytidine, 2'-OMe-adenosine, and 2'-OMe-guanosine are repeated in tandem 7 times from 5' to 3' in this order, consisting of ENA and 2'-O-methylribonucleotide (hereinafter to be referred to as "ENA/2'-OMe ribonucleotide mixmer"). The full length of the mixmer is paired with the CUG trinucleotide repeat sequence in the 3' UTR of RNA of the DMPK gene of myotonic dystrophy type 1 patients. However, since ASO-4 does not contain DNA, it is not degraded by RNase H. The ASO-C of the present invention has the same base sequence as an oligonucleotide consisting of a non-specific sequence used for inhibition experiments with antisense oligonucleotides in other study (Hagemann, TL et al., Ann Neurol, 83:27-39(2018)), and the gap segment of the gapmer is deoxyribonucleotide, which is also common. However, they are different in that the wing region of the gapmer is 2'-O-methoxyethyl (hereinafter to be referred to as "MOE") nucleotide analog in the other study, whereas the wing region of ASO-C used as control in the present specification is ENA.

The following Table 3 is a table comparing the structures of the ASOs of the Example of the present invention and the structures of the ASOs of the related prior art. Since the ASOs of the Example of the present invention have the same base sequences as the ASOs of the related prior art, the base sequences of ASO-1 and PS115 are listed in SEQ ID NO: 11 in the Sequence Listing attached hereto. The base sequences of ASO-2 and ASO-A (ISIS 445569) are listed in SEQ ID NO:12. The base sequences of ASO-3 and PS116 are listed in SEQ ID NO:13. The base sequences of ASO-4 and ASO-B (PS58) are listed in SEQ ID NO:3. The base sequences of ASO-5 and ISIS486178 are listed in SEQ ID NO:14. The base sequence of Control ASO is listed in SEQ ID NO:23.

**[Table 3]**

| | structure | type | structure | SEQ ID NO: |
|---|---|---|---|---|
| ASO-1 | 5'-_{E}G_{E}G_{E}A_{E}G_{E}TdCdGdAdAdGdAdCdAdGdTdT_{E}C_{E}T_{E}A_{E}G_{E}G-3' | RNase H active | ENA/DNA gapmer | SEQ ID NO: 11 |
| PS115 | 5'-dGₛdGₛdAₛdGₛdTₛdCₛdGₛdAₛdAₛdGₛdAₛdCₛdAₛdGₛdTₛdTₛdCₛdTₛdAₛdGₛdG-3' | RNase H active | PT DNA | |
| ASO-2 | 5'-_{E}C_{E}G_{E}G_{E}A_{E}GdCdGdGdTdTdGdTdGdAdA_{E}C_{E}T_{E}G_{E}G_{E}C-3' | RNase H active | ENA/DNA gapmer | SEQ ID NO: 12 |
| ASO-A (ISIS 45569) | 5'-ₘmCₘₛGₘₛGₘₛAₘₛGₛdmCₛdGₛdGₛdTₛdTₛdGₛdTₛdGₛdAₛdAₛₘmCₘₛTₘₛGₘₛGₘₛmC-3' | RNase H active | PT, 5mC, MOE/DNA gapmer | |
| ASO-3 | 5'-_{E}G_{E}G_{E}T_{E}A_{E}CdAdCdAdGdGdAdCdTdGdGdA_{E}G_{E}CT_{E}G_{E}G-3' | RNase H active | ENA/DNA qapmer | SEQ ID NO: 13 |
| PS116 | 5'-dGₛdGₛdTₛdAₛdCₛdAₛdCₛdAₛdGₛdGₛdAₛdCₛdTₛdGₛdGₛdAₛdGₛdCₛdTₛdGₛdG-3' | RNase H active | PT DNA | |
| ASO-4 (ASO-4M) | 5'-_{E}CₘAₘG_{E}CₘAₘG_{E}CₘAₘG_{E}CₘAₘG_{E}CₘAₘG_{E}CₘAₘG_{E}CₘAₘG-3 | blocker | ENA/2'-O-Me RNA Mixmer | SEQ ID NO: 3 |
| ASO-B (PS58) | -5'-ₘCₛₘAₛₘGₛₘCₛₘAₛₘGₛₘCₛₘAₛₘGₛₘCₛₘAₛₘGₛₘCₛₘAₛₘGₛₘCₛₘAₛₘGₛₘCₛₘAₛₘG-3' | blocker | PT 2'-O-Me RNA | |
| ASO-5 | 5'-_{E}A_{E}C_{E}AdAdTdAdAdAdTdAdCdCdG_{E}A_{E}G_{E}G-3' | RNase H active | ENA/DNA qapmer | SEQ ID NO: 14 |
| ISIS 486178 | 5'-ₖAₖCₖAdAdTdAdAdAdTdAdCdCdGₖAₖGₖG-3' | RNase H active | cEt/DNA gapmer | |
| Control ASO | 5'-_{E}CₘA_{E}CₘG_{E}CₘGₘAₘGₘA_{E}CₘA_{E}CₘG_{E}CₘGₘAₘGₘA_{E}CₘGₘA-3' | blocker | ENA/2'-O-Me RNA Mixmer | SEQ ID NO: 23 |

In the structure of each ASO in Table 3, "A", "G", and "C" are adenosine, guanosine, and cytidine, respectively, "dA", "dG", "dC", and "dT" are deoxyadenosine, deoxyguanosine, deoxycytidine, and thymidine, respectively, nucleotide to the right of the subscript "k" is 6'-(S)-CH₃bicyclic nucleotide analog (hereinafter to be referred to as "cEt"),
nucleotide to the right of the subscript "e" is 2'-O-methoxyethyl (hereinafter to be referred to as "MOE") nucleotide analog,
nucleotide to the right of the subscript "E" is ENA, nucleotide to the right of the subscript "m" is 2'-OMe nucleotide analog, and
the subscript "s" shows that a bond between nucleoside to the left and the nucleotide to the right is a phosphorothioate (hereinafter to be referred to as "PT") bond and not a phosphodiester bond. The "mC" is 5 methylcytosine nucleoside.

In Table 3, "uniformly modified" means that means that all nucleotides of the oligonucleotide have the same modified sugar and all the bonds between nucleotides are the same modified bonds.

A prior art related to ASO-1 is PS115 described in Non Patent Literature 7. PS115 is common to ASO-1 in that it has the same base sequence. However, they are different in that all nucleoside bonds between nucleosides are phosphodiester bonds in ASO-1, whereas PT bonds in PS115, and that ASO-1 is a gapmer whose wing region consists of ENA, whereas it is all deoxyribonucleotide in PS115.

A prior art related to ASO-2 is ISIS 445569 described in Non Patent Literatures 5 and 6 (hereinafter also to be referred to as "ASO-A"). ISIS 445569 has the same base sequence as ASO-2, and is common in that it has a gapmer structure consisting of a wing region of the same length. However, they are different in that all nucleoside bonds between nucleosides are phosphodiester bonds in ASO-2, whereas PT bonds in ISIS 445569, that the wing region of gapmer is ENA in ASO-2, whereas MOE-nucleotide analog in ISIS 445569, and that nucleotide at the 5' end and the 3' end is -cytidine -in ASO-2, whereas 5-methylcytidine in ISIS 445569.

A prior art related to ASO-3 is PS116 described in Non Patent Literature 7. PS116 is common to ASO-3 in that it has the same base sequence. However, they are different in that all nucleoside bonds between nucleosides are phosphodiester bonds in ASO-3, whereas PT bonds in PS116, and that ASO-3 is a gapmer whose wing region consists of ENA, whereas it is all deoxyribonucleotide in PS115.

A prior art related to ASO-4 is PS58 described in Non Patent Literatures 7 and 8. PS58 (hereinafter also to be referred to as "ASO-B") is common to ASO-4 in that it has the same base sequence and all adenosine nucleotide and guanosine nucleotide are OMe nucleotide analogs. However, they are different in that cytidine nucleotide is also OMe nucleotide analog in PS58, whereas ENA in ASO-4. They are also different in that all nucleoside bonds between nucleosides are PT bonds in PS58, whereas phosphodiester bonds in ASO-4.

A prior art related to ASO-5 is ISIS 486178 described in Non Patent Literature 6. ISIS 486178 is common to ASO-2 in that it has the same base sequence, and it has a gapmer structure consisting of a wing-region of the same length. However, they are different in that ASO-5 is an ENA/DNA gapmer, whereas ISIS 486178 is a gapmer whose wing region consists of 6'-(S)-CH₃bicyclic (cEt) nucleotide and the gap region consists of unmodified deoxyribonucleotide (hereinafter to be referred to as "cEt/DNA gapmer").

The set of primers used for triplet repeat prime PCR (hereinafter to be referred to as "TP-PCR") analysis is shown in Table 4. The base sequences of each primer are listed in SEQ ID NOs: 15 to 22 in the Sequence Listing attached to the present specification.

**[Table 4]**

| | primer name | sequence | SEQ ID NO: |
|---|---|---|---|
| TP-PCR forward primer | FAM-P1-F | 5'FAM-GGGGC'l'CGAAGGGTCCTTGT-3' | SEQ ID NO:15 |
| | P2-R | 5'-GTGCGTGGAGGATGQAACACG-3' | SEQ ID NO:16 |
| | P3 | 5'-AGCGGATAACAATTTCACACAGGA-3' | SEQ ID NO:17 |
| | P4-(CAG)₆-R | 5'-AGCGGATAACAATTTCACACAGGACAGCAGCAGCAGCAGCAG-3' | SEQ ID NO: 18 |
| TP-PCR reverse | P1-F | 5'-GGGGCTCGAAGGGTCCTTGT-3' | SEQ ID NO:19 |
| | HEX-P2-R | 5'Hex-GTGCGTGGAGGATGGAACACG-3' | a SEQ ID NO:20 |
| | P3 | 5'-AGCGGATAACAATTTCACACAGGA-3' | SEQ ID NO:21 |
| | P4(CTG)6-F | 5'-AGAGGATAACAATTTCACACAGGATGCTGCTGCTGCTGCTGCTG-3' | SEQ ID NO: 22 |

"FAM-" in the sequence of primer FAM-P1-F in Table 4 means that 6-carboxyfluorescein is covalently bonded to the 5' end of the primer oligonucleotide, and "HEX-" in the sequence of primer HEX-P2-R in Table 4 means that hexachlorofluorescein is covalently bonded to the 5' end of the primer oligonucleotide.

The oligonucleotide according to the present invention has a mixmer structure that is not cleaved by RNase H. As used herein, the "mixmer structure" is a structure of a single-stranded oligonucleotide in which RNase H active nucleotides and RNase H inactive nucleotides are mixed. A hetero double strand nucleic acid formed by pairing the single-stranded oligonucleotide with complementary RNA refers to a structure that is not degraded by RNase H. According to Monia, B.P. et al. (J. Biological. Chem., 268: 14514 (1993)), the degradation activity by RNase H against a hetero double strand nucleic acid composed of a gapmer ASO in which a gap region consisting of at least 5 consecutive RNase H active nucleotides is sandwiched between wing regions of RNase H inactive nucleotides, and an RNA complementary to the ASO exceeded 75%, but the degradation activity by RNase H against a hetero double strand nucleic acid composed of a gapmer ASO in which a gap region consisting of not more than 4 consecutive RNase H active nucleotides is sandwiched between wing regions of RNase H inactive nucleotides, and an RNA complementary to the ASO was less than 20%. Therefore, the oligonucleotide with the mixmer structure of the present invention may have only 5 or 4 or less, for example, 5, 4, 3, or 2 consecutive RNase H-activated nucleotides. The "mixmer ASO" or "ASO mixmer" refers to an ASO having a mixmer structure.

In the present invention, whether a specific type of nucleotide analog is of RNase H active type or RNase H inactive type can be determined, for example, by the following procedure. An oligonucleotide with a gapmer structure in which a gap region with 10 consecutive nucleotide analogs of the specific type is sandwiched between known RNase H active nucleotides, for example, wing regions consisting of five 2'-OMe-nucleotide analogs is generated. A hetero double strand complex is prepared by pairing 300 picomoles of an oligonucleotide with the gapmer structure with 1500 picomoles of RNA to be paired with the oligonucleotide. The hetero double strand complex and 3.0 units of Escherichia coli RNase H are added to a reaction solution for RNase H, for example, a reaction solution consisting of 50 mM Tris-HCl (pH 8.0), 75 mM KCl, 3 mM MgCl₂, and 10 mM dithiothreitol, to make a total volume of 150 µL, reacted at 37°C for 60 min, and the reaction product is analyzed by an agarose gel electrophoresis method.

In addition, those skilled in the art can determine whether a specific type of nucleotide analogue is RNase H active or RNase H inactive. For example, EP 1 222 309 provides an in vitro method for determining RNase H activity that can be used to determine the ability to recruit RNase H.

The nucleoside monomers of the oligonucleotides contained in the pharmaceutical composition of the present invention are linked together via internucleoside linking groups. Each monomer is appropriately linked to the 3' adjacent monomer via a linking group. The terminal 5' monomer of the oligonucleotide may or may not contain a 5' end group or a linking group for conjugation, but does not contain a 5' linking group.

The terms "linking group" and "internucleotide linkage" refer to a group that can link two nucleosides with a covalent bond to form a dinucleotide. Natural nucleotides are linked by a phosphodiester bond via a phosphate group. Non-natural, that is, modified nucleotide bonds include, but are not limited to, phosphorothioate groups, phosphorodithioate groups, and boranophosphate groups. Internucleoside linkage may be used interchangeably with internucleotide linkage. Generally, oligonucleotides containing modified nucleotide bonds have higher membrane permeability and higher resistance to intracellular and extracellular nucleases than oligonucleotides linked only by phosphodiester bonds. On the other hand, a hetero double strand nucleic acid formed by hybridizing an oligonucleotide containing a modified nucleotide bond with RNA in a cell is degraded by RNase H in the same way as hetero double strand nucleic acids formed by hybridizing an oligonucleotide linked only by phosphodiester bonds with RNA in a cell. In addition, modified nucleotide bonds have stereoisomers about the sulfur atom, for example, phosphorothioate groups. The oligonucleotide contained in the pharmaceutical composition of the present invention may be synthesized such that modified nucleotide bonds of a specific stereoisomer are used for specific internucleotide linkage of the oligonucleotide, or may be synthesized such that a part or all of the internucleotide linkages is/are racemate(s).

In one embodiment of the pharmaceutical composition of the present invention, the aforementioned oligonucleotide may contain
(1) an oligonucleotide consisting of a base sequence of any of SEQ ID NOs: 1 to 9,
(2) an oligonucleotide consisting of a base sequence consisting of adenine-guanine, a base sequence of any of SEQ ID NOs: 1 to 9, and cytosine, from the 5' end to the 3' end, or
(3) an oligonucleotide consisting of a base sequence consisting of guanine, a base sequence of any of SEQ ID NOs: 1 to 9, and cytosine-adenine, from the 5' end to the 3' end.

The first oligonucleotide in the aforementioned embodiment of the pharmaceutical composition of the present invention, that is, "an oligonucleotide consisting of a base sequence of any of SEQ ID NOs: 1 to 9" is an oligonucleotide consisting of a base sequence with a repeat number of 5 - 13 of a trinucleotide in which cytidine, adenosine, and guanosine are linked in order from the 5' end to the 3' end.

The second oligonucleotide in the aforementioned embodiment of the pharmaceutical composition of the present invention, that is, "an oligonucleotide consisting of a base sequence consisting of adenine-guanine, a base sequence of any of SEQ ID NOs: 1 to 9, and cytosine, from the 5' end to the 3' end" is an oligonucleotide consisting of a base sequence with a repeat number of 6 - 14 of a trinucleotide in which adenosine, guanosine, and cytidine are linked in order from the 5' end to the 3' end.

The third oligonucleotide in the aforementioned embodiment of the pharmaceutical composition of the present invention, that is, "an oligonucleotide consisting of a base sequence consisting of guanine, a base sequence of any of SEQ ID NOs: 1 to 9, and cytosine-adenine, from the 5' end to the 3' end" is an oligonucleotide consisting of a base sequence with a repeat number of 6 - 14 of a trinucleotide in which guanosine, cytidine, and adenosine are linked in order from the 5' end to the 3' end.

In one embodiment of the pharmaceutical composition of the present invention, the aforementioned RNase H inactive nucleotide analog may contain a nucleotide analog modified at the 2'-position of ribose and/or a modified nucleotide analog bridged between the 2'-position and the 4'-position of ribose.

A nucleotide analog modified at the 2'-position of ribose which is contained in the oligonucleotide in the aforementioned embodiment includes, but is not limited to, 2'-F nucleotide analog, 2'-OMe-nucleotide analog, MOE-nucleotide analog, 2'-(3-hydroxy)propylnucleotide analog, and 2'-AP nucleotide analog.

The aforementioned nucleotide analogs modified at the 2'-position of ribose is represented by, for example, the following chemical formula (1) wherein substituent X to be bonded to the 2'-position of ribose is selected from a fluoro group, a methoxy group, an O-methoxyethyl group, a 2'-(3-hydroxy)propyl group, and a 2'-(3-amino)propyl group, Z and Z* are each independently selected from an internucleotide linkage, R, a terminal group, and a protecting group, B is selected from natural or non-natural nucleotide base moieties (nucleic acid bases), and R is selected from hydrogen, a hydroxyl group, a C₁₋₄ alkyl group, and a C₁₋₄ alkoxy group.

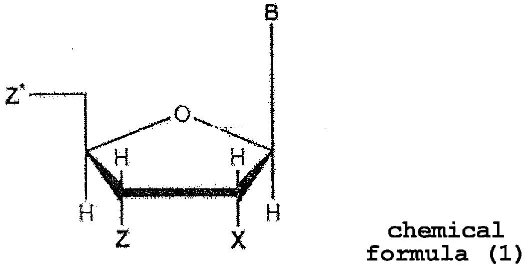

In chemical formula (1), a nucleotide analog in which substituent X to be bonded to the 2'-position of ribose is a fluoro group is a 2'-F nucleotide analog, a nucleotide analog in which substituent X to be bonded to the 2'-position of ribose is a methoxy group is a 2'-OMe-nucleotide, analog, a nucleotide analog in which substituent X to be bonded to the 2'-position of ribose is an O-methoxyethyl group is a MOE-nucleotide analog, a nucleotide analog in which substituent X to be bonded to the 2'-position of ribose is a 2'-(3-hydroxy)propyl group is a 2'-(3-hydroxy)propylnucleotide analog, and a nucleotide analog in which substituent X to be bonded to the 2'-position of ribose is a 2'-(3-amino)propyl group is a 2'-AP nucleotide analog.

A modified nucleotide analog bridged between the 2'-position and the 4'-position of ribose to be contained in the oligonucleotide of the aforementioned embodiment includes, but is not limited to, oxy-LNA, thio-LNA, amino-LNA, 5'-methyl-LNA, ENA, cEt, and cMOE.

The aforementioned modified nucleotide analog bridged between the 2'-position and the 4'-position of ribose is also referred to as locked nucleic acid or LNA, because the conformation of the 2'-position and the 4'-position is fixed (locked). Alternatively, it is also referred to as BNA or bicyclic nucleic acid because it is crosslinked (bridged). The aforementioned modified nucleotide analog bridged between the 2'-position and the 4'-position of ribose includes oxy-LNA, thio-LNA, amino-LNA, 5'-methyl-LNA, ENA, cEt, and cMOE. Among these, oxy-LNA, thio-LNA, amino-LNA, 5'-methyl-LNA, and ENA have stereoisomers of β-D-configuration and α-L-configuration, and cEt and cMOE have R and S stereoisomers having the carbon atom bridging between the 2'-position and the 4'-position of the aforementioned ribose as the asymmetric center.

The aforementioned modified nucleotide analog bridged between the 2'-position and the 4'-position of ribose has, for example, a structure of the following chemical formulas (2) and (3) wherein a divalent atomic group "-Y-" to be bonded to the 2'-position of ribose is selected from "-O-", "-S-", "-CH₂-S-", "-N(H)-", "-N(R^{a})-", "-CH₂-N(H)-", and "-CH₂-N(R^{a})-", Z and Z* are each independently selected from an internucleotide linkage, R^{b}, a terminal group, and a protecting group, B is selected from natural or non-natural nucleotide base moieties (nucleic acid bases), R^{a} is independently selected from hydrogen and C₁₋₄ alkyl, and R^{b} is selected from hydrogen, a hydroxyl group, a C₁₋₄ alkyl group, and a C₁₋₄ alkoxy group.

The oxy-LNA, thio-LNA, and amino-LNA with β-D-configuration are represented by the formula (2), and oxy-LNA, thio-LNA, and amino-LNA with α-L-configuration are represented by the formula (3). In the following chemical formulas (2) and (3), a nucleotide analog in which a divalent atomic group "-Y-" to be bonded to the 2'-position of ribose is "-O-" is oxy-LNA, a nucleotide analog in which a divalent atomic group "-Y-" to be bonded to the 2'-position of ribose is "-S-" or "-CH₂-S-" is thio-LNA, and a nucleotide analog in which a divalent atomic group "-Y-" to be bonded to the 2'-position of ribose is selected from "-N(H)-", "-N(R^{a})-", "-CH₂-N(H)-", and "-CH₂-N(R^{a})-" (wherein R^{a} is selected from C₁₋₄ alkyl) is amino-LNA.

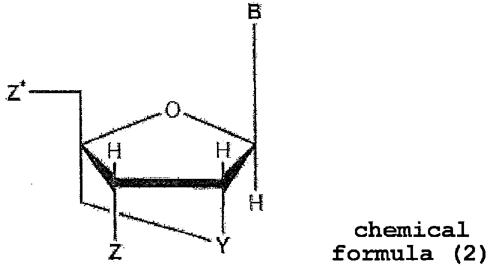

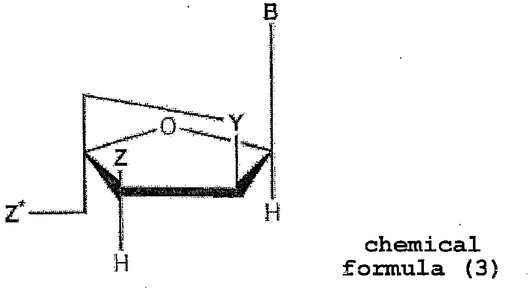

The 5'-methyl-LNA with β-D-configuration is represented by the following chemical formula (4), and 5'-methyl-LNA with α-L-configuration is represented by the following chemical formula (5).

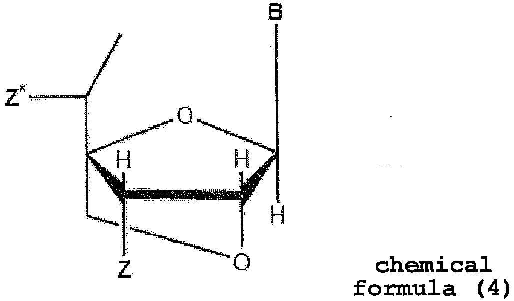

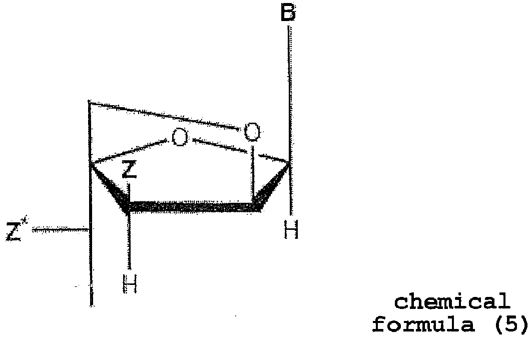

The ENA with β-D-configuration is represented by the following chemical formula (6), and ENA with α-L-configuration is represented by the following chemical formula (7).

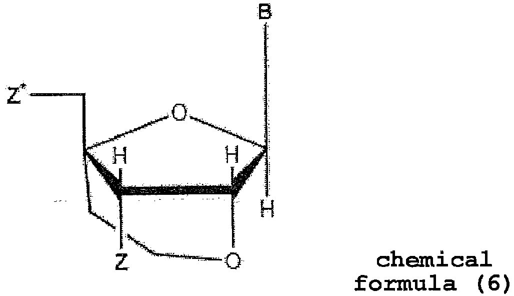

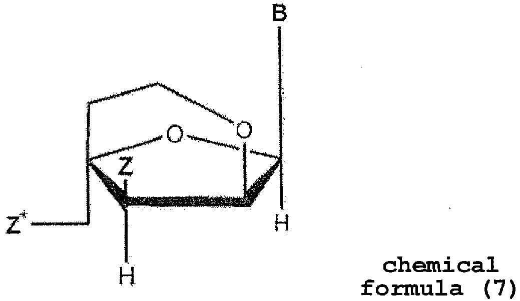

R-type cEt and cMOE are represented by the following formula (8), and S-type cEt and cMOE are represented by the following formula (9). In the structures of the chemical formulas (8) and (9), W is selected from a methyl group and a methoxymethyl group, Z and Z* are independently selected from an internucleotide linkage, R (R is selected from hydrogen, hydroxyl group, C₁₋₄ alkyl group, and C₁₋₄ alkoxy group), a terminal group, and a protecting group, and B is selected from natural or non-natural nucleotide base moieties (nucleic acid bases).

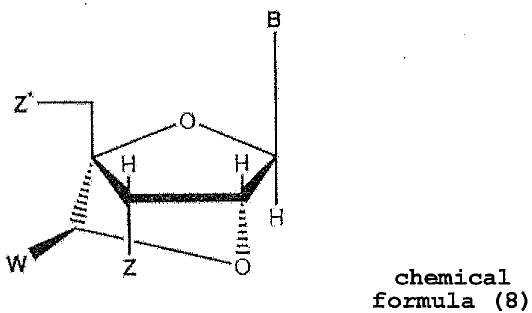

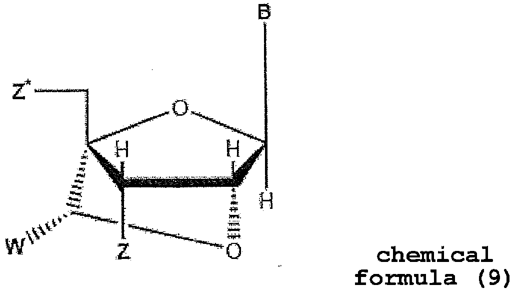

In the chemical formulas (8) and (9), a nucleotide analogue in which W is a methyl group is cEt, and a nucleotide analogue in which W is a methoxymethyl group is cMOE.

In one embodiment of the pharmaceutical composition of the present invention, the aforementioned nucleotide analog with a modified oxygen atom at the 2'-position of ribose may contain a 2'-OMe-nucleotide analog and/or a MOE-nucleotide analog.

In one embodiment of the pharmaceutical composition of the present invention, the aforementioned modified nucleotide analog bridged between the 2'-position and the 4'-position of ribose may contain β-D-oxy-L-LNA, β-D-ENA and/or R-type cEt.

In one embodiment of the pharmaceutical composition of the present invention, all the aforementioned nucleotide analogs modified at the 2'-position of ribose may contain the same modified sugar.

In one embodiment of the pharmaceutical composition of the present invention, the aforementioned nucleotide analog modified at the 2'-position of ribose may have at least two different modified sugars.

In one embodiment of the pharmaceutical composition of the present invention, all the aforementioned modified nucleotide analogs bridged between the 2'-position and the 4'-position of ribose may contain the same modified sugar.

In one embodiment of the pharmaceutical composition of the present invention, the aforementioned modified nucleotide analog bridged between the 2'-position and the 4'-position of ribose may have at least two different modified sugars.

In one embodiment of the pharmaceutical composition of the present invention, the aforementioned nucleotide analog with a modified oxygen atom at the 2'-position of ribose to be contained in the aforementioned oligonucleotide may be a 2'-OMe-nucleotide analog and/or a MOE-nucleotide analog.

The aforementioned 2'-OMe-nucleotide analog is a nucleotide analog of the aforementioned chemical formula (1) wherein the substituent X bonded to the 2'-position of ribose is a methoxy group and base B is adenine, guanine, thymine, or cytosine. The aforementioned MOE-nucleotide analog is a nucleotide analog of the aforementioned chemical formula (1) wherein the substituent X bonded to the 2'-position of ribose is an O-methoxyethyl group and base B is adenine, guanine, thymine, or cytosine.

In one embodiment of the pharmaceutical composition of the present invention, the aforementioned modified nucleotide analog bridged between the 2'-position and the 4'-position of ribose to be contained in the aforementioned oligonucleotide may be LNA, ENA and/or cEt.

In one embodiment of the pharmaceutical composition of the present invention, all the aforementioned nucleotide analogs modified at the 2'-position of ribose may contain the same modified sugar. That is, in the aforementioned nucleotide analog modified at the 2'-position of ribose to be contained in the aforementioned oligonucleotide may be any one kind of, the substituent bonded to the 2'-position of ribose may be any one kind of a fluoro group, a methoxy group, or a O-methoxyethyl group.

In one embodiment of the pharmaceutical composition of the present invention, the aforementioned nucleotide analog modified at the 2'-position of ribose may have at least two different modified sugars. That is, in the aforementioned nucleotide analog modified at the 2'-position of ribose to be contained in the aforementioned oligonucleotide may have 2 kinds or 3 kinds of substituents selected from the group consisting of a fluoro group, a methoxy group, and an O-methoxyethyl group.

In one embodiment of the pharmaceutical composition of the present invention, all the aforementioned modified nucleotide analogs bridged between the 2'-position and the 4'-position of ribose may have the same modified sugar. That is, the aforementioned modified nucleotide analog bridged between the 2'-position and the 4'-position of ribose to be contained in the aforementioned oligonucleotide may be any one kind of LNA, ENA or cEt.

In one embodiment of the pharmaceutical composition of the present invention, the aforementioned modified nucleotide analog bridged between the 2'-position and the 4'-position of ribose may have at least two different modified sugars. That is, in the aforementioned modified nucleotide analog bridged between the 2'-position and the 4'-position of ribose to be contained in the aforementioned oligonucleotide may have 2 kinds or 3 kinds of substituents selected from the group consisting of LNA, ENA, and cEt.

In one embodiment of the pharmaceutical composition of the present invention, the aforementioned oligonucleotide may contain β-D-ENA-cytidine, 2'-OMe-adenosine, and 2'-OMe-guanosine.

In one embodiment of the pharmaceutical composition of the present invention, the cytidines-in the aforementioned oligonucleotide may all be β-D-ENA-cytidines, adenosines may all be 2'-OMe-adenosines, and guanosines may all be 2'-OMe-guanosines.

In one embodiment of the pharmaceutical composition of the present invention, the oligonucleotide to be contained in the pharmaceutical composition of the present invention may contain at least one type of nucleotide bond selected from the group consisting of phosphorothioate, phosphorodithioate, and boranophosphate.

In one embodiment of the pharmaceutical composition of the present invention, the pharmaceutical composition of the present invention may contain a pharmaceutically acceptable carrier or diluent. As the carrier to be contained in the pharmaceutical composition of the present invention, those of ordinary skill in the art can select a carrier suitable for the situation. Carriers that can be selected include, but are not limited to, preservatives such as sodium benzoate, sodium hydrogen sulfite, methylparaben, propylparaben, and the like, pH adjusters such as sodium dihydrogenphosphate, anhydrous sodium monohydrogen phosphate, citric acid, sodium citrate, and the like, isotonicity agents such as glucose, sodium chloride, potassium chloride, and the like, divalent ion regulators such as calcium chloride, magnesium chloride, and the like. These carriers are not limited to use for the purpose of exhibiting a single action, but can be used for the purpose of exhibiting multiple actions. Diluents that can be contained in the pharmaceutical composition of the present invention include, but are not limited to, liquid, for example, phosphate buffered saline.

In one embodiment of the pharmaceutical composition of the present invention, the pharmaceutical composition of the present invention may contain a carrier for cell membrane permeation and/or intranuclear delivery, and the carrier for cell membrane permeation and/or intranuclear delivery can be bonded to or associated with the oligonucleotide in the pharmaceutical composition of the present invention.

The aforementioned carrier for cell membrane permeation and/or intranuclear delivery may contain peptide. Peptides as carrier for cell membrane permeation include, but are not limited to, cell membrane penetrating peptides such as oligoarginines such as octaarginine and the like, basic peptides derived from Tat protein of HIV-1, basic helical peptides derived from the Antennapedia homeodomain protein of Drosophila, and the like. Peptides as the aforementioned carrier for intranuclear delivery include, but are not limited to, peptides that are nuclear localization signals that specifically bind to importin and are derived from SV40 virus large T antigen, c-myc, nucleoplasmin, and the like.

The aforementioned carrier for cell membrane permeation and/or intranuclear delivery may contain peptide mimetics. The peptide mimetics as a carrier for cell membrane permeation and/or intranuclear delivery is a synthetic compound mimicking the structure of peptide as the aforementioned carrier for cell membrane permeation and/or intranuclear delivery. It binds to or associates with the oligonucleotide in the pharmaceutical composition of the present invention and promotes cell membrane permeation and/or nuclear delivery. The peptide mimetics as a carrier for cell membrane permeation and/or intranuclear delivery is a compound in which the peptide as the aforementioned carrier for cell membrane permeation and/or intranuclear delivery is entirely or partially substituted with compounds other than natural peptides, such as heterocycles, cyclic compounds, unnatural amino acids, D-amino acids, and equivalents of various functional groups.

The carrier for cell membrane permeation and/or intranuclear delivery may contain cationic polymers of DEAE dextran, polyethyleneimine (PEI), and polypropylene imine (PPI).

The carrier for cell membrane permeation and/or intranuclear delivery may contain a lipid. Lipids as the carrier for cell membrane permeation and/or intranuclear delivery may contain, without limitation, phospholipids such as 1,2-dimyristoyl-sn-glycero-3-phosphatidylcholine (DMPC), 1,2-dipalmitoyl-sn-glycero-3-phosphatidylcholine (DPPC), 1,2-distearoyl-SN-glycero-3-phosphatidylcholine (DSPC), 1-palmitoyl-2-myristoyl-SN-glycero-3-phosphatidylcholine (PMPC), 1-stearoyl-2-myristoyl-SN-glycero-3-phosphatidylcholine (SMPC), soybean-derived hydrogenated lecithin (HSPC), 1-stearoyl-2-oleyl-sn-glycero-3-phosphatidylcholine (SOPC), 1-palmitoyl-2-oleyl-sn-glycero-3-phosphatidylcholine (POPC), 1,2-dioleoyl-sn-glycero-3-phosphocholine (DOPC), 1,2-dioleoyl-SN-glycero-3-phosphoglycerol (DOPG), 1,2-dioleoyl-SN-glycero-3-phospho-L-serine (DOPS), 1,2-dioloyloyl-SN-glycero-3-phosphoric acid (DOPA), 1,2-dipalmitoyl-sn-glycero-3-phosphocholine (DPPC), 1,2-dimyristoyl-sn-glycero-3-phospho-rac-(1-glycerol) (DMPG), 1,2-dioleyl-sn-glycero-3-phospho ethanolamine (DOPE), and the like, anionic lipids such as 1,5-O-dihexadecyl-N-succinyl-L-glutamic acid and the like, cholesterol, PEG-conjugated lipids such as N-(carbonylmethoxypolyethylene glycol 2000)-1,2-distearoyl-SN-glycero-3-phosphoethanolamine and the like, and pH sensitive lipids such as 1,2-dioleyl-3-dimethylammonium propane (DODAP).

The carrier for cell membrane permeation and/or intranuclear delivery may also contain a lipitoid. Lipitoids as the aforementioned carrier for cell membrane permeation and/or intranuclear delivery refers to a synthetic compound in which peptide and/or peptide mimetics as the aforementioned carrier for cell membrane permeation and/or intranuclear delivery, and lipid as the aforementioned carrier for cell membrane permeation and/or intranuclear delivery are linked by a covalent bond.

The carrier for cell membrane permeation and/or intranuclear delivery may also contain a liposome. Liposome as the aforementioned carrier for cell membrane permeation and/or intranuclear delivery refers to a lipid membrane structure dispersed in an aqueous solvent and enclosing the aqueous solvent inside. The lipid membrane of the liposome may be a single-layer or multi-layer lipid bilayer membrane or lipid multilayer membrane. Liposomes may be particulate or reticulated. Lipid components of liposomes may include, but are not limited to, lipids as the aforementioned carriers for cell membrane permeation and/or intranuclear delivery. Lipid components of liposomes may contain lipitoid as the aforementioned carrier for cell membrane permeation and/or intranuclear delivery. The liposome as the aforementioned carrier for cell membrane permeation and/or intranuclear delivery can encapsulate an oligonucleotide according to the pharmaceutical composition of the present invention therein.

The carrier for cell membrane permeation and/or intranuclear delivery may also contain a lipid nanoparticle (LNP). Lipid nanoparticles as the aforementioned carrier for cell membrane permeation and/or intranuclear delivery is composed of the same lipid as the lipid component of the aforementioned liposomes and the oligonucleotide in the pharmaceutical composition of the present invention. As the lipid nanoparticle, pH sensitive lipids such as 1,2-dioleyl-3-dimethylammonium propane (DODAP) that are positively charged in low pH environment may be used. Unlike liposomes, lipid nanoparticles have a single lipid membrane and have a core structure filled with lipid instead of enclosing an aqueous solvent inside. Lipid nanoparticles as the aforementioned carrier for cell membrane permeation and/or intranuclear delivery may contain the oligonucleotide in the pharmaceutical composition of the present invention in the aforementioned core structure.

In one embodiment of the pharmaceutical composition of the present invention, the pharmaceutical composition of the present invention can be delivered by at least one parenteral administration selected from the group consisting of subcutaneous administration, intravenous administration, intraarterial administration, intramuscular administration, intraperitoneal administration,intracranial administration, and intrathecal administration. The aforementioned administration may be continuous or chronic, short term, or intermittent. The pharmaceutical composition of the present invention results in downregulation of CUG-RNA aggregate formation for at least 30 days, at least 35 days, at least 40 days, at least 45 days, at least 50 days, at least 55 days, at least 60 days, at least 65 days, at least 70 days, at least 75 days, at least 80 days, at least 85 days, at least 90 days, at least 95 days, at least 100 days, at least 105 days, at least 110 days, at least 115 days, at least 120 days, or at least 1 year after administration, and/or 50%, 55%, 60%, 65%, 70%, 75%, 80%, 85%, 90%, 95% or 100% reduction in intracellular levels of CUG-RNA or DMPK protein.

The dosage form of the pharmaceutical composition of the present invention may be oral administration such as tablet, capsule, granule, powder, syrup, or the like. These preparations are produced by well known methods using additives such as excipients (e.g., organic excipients such as sugar derivatives such as lactose, sucrose, glucose, mannitol, sorbitol; starch derivatives such as cornstarch, potato starch, α-starch, dextrin; cellulose derivatives such as crystalline cellulose; gum arabic; dextran; pullulan: and inorganic excipients such as silicate derivatives such as light silicic anhydride, synthetic aluminum silicate, calcium silicate, magnesium aluminometasilicate; phosphates such as calcium hydrogen phosphate; carbonates such as calcium carbonate; sulfates such as calcium sulfate and the like), lubricants (e.g., stearic acid metal salts such as stearic acid, calcium stearate, magnesium stearate; talc; colloid silica; waxes such as veegum, spermaceti; boric acid; adipic acid; sulfates such as sodium sulfate; glycol; fumaric acid; sodium benzoate; DL leucine; fatty acid sodium salt; lauryl sulfates such as sodium lauryl sulfate, magnesium lauryl sulfate; silicic acids such as silicic anhydride, silicic acid hydrate; and the above-mentioned starch derivative), binders (e.g., hydroxypropylcellulose, hydroxypropylmethylcellulose, polyvinylpyrrolidone, macrogol, and compounds similar to the aforementioned excipients), disintegrants (e.g., cellulose derivatives such as low-substituted hydroxypropylcellulose, carboxy group methylcellulose, carboxy group methylcellulose calcium, internally crosslinked carboxy group methylcellulose sodium; chemically-modified starch and celluloses such as carboxy group methylstarch, carboxy group methylstarch sodium, crosslinked polyvinylpyrrolidone), stabilizers (p-hydroxybenzoic acid esters such as methylparaben, propylparaben; alcohols such as chlorobutanol, benzyl alcohol, phenylethylalcohol; benzalkonium chloride; phenols such as phenol, cresol; thimerosal; dehydroacetic acid; and sorbic acid), flavoring agents (e.g., sweetener, acidulant, flavor and the like used generally) and the like.

The active ingredient contained in the pharmaceutical composition of the present invention is the aforementioned oligonucleotide (when the oligonucleotide is bound or associated with a carrier for cell membrane penetration and/or intranuclear delivery, the aforementioned oligonucleotide bound or associated with the aforementioned carrier for cell membrane penetration and/or intranuclear delivery). The ratio of the active ingredient contained in the pharmaceutical composition of the present invention can be appropriately determined within the range where the desired effect can be afforded. It is generally 0.01 - 100 wt%, preferably 0.1 - 99.9 wt%, more preferably 0.5 - 99.5 wt%.

The dosage for administering the pharmaceutical composition of the present invention must be carefully adjusted in consideration of the age, body weight, and condition of the individual to be treated, administration route, dosage form, and administration method, and the exact dosage must be determined by a physician. The actual dosage is within the discretion of the physician and may vary taking into account the particular situation of the present invention to afford the desired therapeutic effect. The dose of the pharmaceutical composition of the present invention is not generally defined in view of the kind of the aforementioned active ingredient, and the body weight, age, symptoms and the like of the administration subject. In the case of, for example, parenteral administration, it is desirable to administer 0.001 mg/kg body weight (preferably 0.01 mg/kg body weight) as the lower limit and 100 mg/kg body weight (preferably 10 mg/kg body weight) as the upper limit per administration, and in the case of one oral administration, it is desirable to administer 0.01 mg/kg body weight (preferably, 0.1 mg/kg body weight) as the lower limit and 1000 mg/kg body weight (preferably, 100 mg/kg body weight) as the upper limit per administration, each once to several times a day, depending on the symptoms.

The administration subject of the pharmaceutical composition of the present invention is a patient with myotonic dystrophy type 1 disease. In the Examples of the present invention, DM-1 and 3 cells showed a more remarkable effect of eliminating RNA aggregates than DM-2 cells. Thus, patients with higher repeat number of the CTG sequence of the DMPK gene are preferred as the administration subject of the pharmaceutical composition of the present invention.

As the desirable therapeutic effects used in determining the selection of the active ingredient, carrier, dosage, and administration method of the pharmaceutical composition of the present invention, suppression and/or reduction of the progression of clinical symptoms of myotonic dystrophy type 1 disease (myotonic phenomenon (prolonged state of muscle contraction), muscle weakness or atrophy, cataract, retinal degeneration, cardiac conduction disorder, cardiac muscle disorder, gastrointestinal tract symptoms such as swallowing, constipation, and diarrhea associated with smooth muscle disorder, higher brain dysfunction, glucose tolerance disorder, hyperinsulinemia, diabetes, endocrine disorders such as abnormal secretion of growth hormone, and skin symptoms such as pilomatorixoma and epithelioma) are/is most important. However, pharmacodynamic biomarkers (DMPK RNA aggregate formation and/or reduction and/or elimination of deregulation of RNA-binding molecules, including CUG-BP1 and MBNL1) can also be used as surrogate endpoints prior to clinical symptoms, when determining the selection of the active ingredient, carrier, dosage and administration method of the pharmaceutical composition of the present invention.

The pharmaceutical composition of the present invention can be delivered by any one or two or more parenteral administrations selected from the group consisting of transdermal administration, intraocular administration, intra-lens administration, gastrointestinal tract intramucosal administration, gastrointestinal tract submucosal administration, subcutaneous administration, intravenous administration, intraarterial administration, intramuscular administration, intraperitoneal administration, intracranial administration, and intrathecal administration. In addition, the pharmaceutical composition of the present invention can be administered, according to the clinical symptoms of patients, to target tissues including, but not limited to, skeletal muscle, cardiac muscle, smooth muscle, satellite cell, ocular tissues including lens, retina, and the like, gastrointestinal tract from esophagus to colon, nerves of central nervous system and peripheral nervous system, pancreas, adrenal gland, pituitary gland and endocrine tissues involved in the control of these, skin tissue, and the like.

In one embodiment of the assay system of the pharmaceutical composition of the present invention, the assay system of the present invention includes undifferentiated iPS cells established from a human suffering from myotonic dystrophy type 1 disease and a compound to be the evaluation target. The compound to be the evaluation target includes oligonucleotides that target the RNA of myotonic dystrophy protein kinase in myotonic dystrophic type 1 disease.

In the present specification, the adnominal "about" that modifies numerical values means a numerical range of 90% or more and 110% or less of the numerical values. For example, "about 40 bases" refers to bases in a numerical range of 36 bases or more and 44 bases or less.

All documents referred to in the present specification are hereby incorporated by reference in their entirety.

The examples of the present invention described below are for illustrative purposes only and do not limit the technical scope of the present invention. The technical scope of the present invention is limited only by the description of the CLAIMS. Modifications of the present invention, such as addition, deletion, and substitution of constituent elements of the present invention, can be made without departing from the gist of the present invention.

### [Example]

### Example 1 Establishment of iPS cells derived from myotonic dystrophy patient

### (1.1) material and method

Using peripheral blood mononuclear cells (PBMC) cells collected from 3 patients with myotonic dystrophy type 1 in the experimental group and from 2 healthy subjects in the control group, and dermal fibroblasts collected from 1 healthy subject in the control group as starting cells, iPS cells were generated by reprogramming with episomal vectors encoding SOX2, KLF4, OCT4, L-MYC, LIN28, p53 carboxyl terminal dominant negative fragment, and EBNA1 (Okita, K. et al., Stem Cells, 31:458-466(2013)). Cell line authenticity was confirmed by STR analysis using PowerPlex (registered trade mark) 16 system (Promega, Madison, Wisconsin, USA). Karyotype and Southern blot analysis were performed by LSI Medience Corporation (Tokyo). iPS cells were cultured and maintained in StemFit (registered trade mark) medium (AK02N, Ajinomoto Co., Inc., Tokyo) explained by Nakagawa, M. et al. (Sci Rep, 4: 3594 (2014)) on a culture vessel coated with iMatrix-511 (892012, Nippi, Incorporated, Tokyo).

Analysis by TP-PCR in the Examples in the present specification was performed as follows. Genomic DNA of iPS cells was extracted using the PureLink (trademark) Genomic DNA Mini Kit (Invitrogen, Thermo Fisher Scientific, Waltham, Massachusetts, USA), and subjected to TP-PCR analysis described in Singh, S. et al. (Front Genet, 5: 94(2014)) and Non Patent Literature 1 (Chakraborty, S. et al., Current Protocols in Human Genetics, 91: 9.29.1-9.29.19. (2016)). The base sequences of the primers used for TP-PCR analysis are shown in the above-mentioned Table 4.

Immunocytochemical analysis in the Examples in the present specification was performed as follows. Cells were fixed in PBS containing 4% paraformaldehyde (NACALAI TESQUE, INC., Kyoto) for 20 min at room temperature and incubated in a blocking buffer (PBS containing 5% Blocking One (NACALAI TESQUE, INC.) and 0.2% Triton-X100 (NACALAI TESQUE, INC.)). A list of sources and dilution ratios for the primary antibody and the secondary antibody used in the Examples in the present specification is shown in the following Table 5. The primary antibody was reacted with the aforementioned cells in the aforementioned blocking buffer at 4°C for 16 hr and the secondary antibody was reacted at room temperature for 1 hr. Nuclei were stained with DAPI. Images were acquired using the Opera Phenix (trademark) High Content Screening System (PerkinElmer, Waltham, Massachusetts) or a BZ-X710 microscope (KEYENCE, Osaka, Japan).

**[Table 5]**

| antibody name | dilution ratio | source, catalog No. |
|---|---|---|
| rabbit anti-NANOG | 1: 500 | Cosmo Bio, Cat# RCAB0003P, RRID: AB 1962353 |
| mouse anti-OCT4 | 1: 1,000 | Santa Cruz Biotechnology, Cat# sc-5279, RRID: AB 628051 |
| mouse anti-SSEA4 (stage-specific embryonic antigen-4) | 1: 1,000 | Millipore, Cat# MAB4304, RRID: AB_177629 |
| mouse anti-TRA-1-60 | 1: 1,000 | Cell Signaling Technology, Cat# 4746, RRID: AB 2119059 |
| mouse anti-tubulin βIII (TUJI) | 1: 2,000 | Millipore, Cat# MAB1637, RRID: AB 2210524 |
| mouse anti-tubulin βIII (TUJI) | 1: 2,000 | Cell Signaling Technology, Cat# 5568, RRID: AB 10694505 |
| goat anti-SOX17 | 1: 1,000 | R&D Systems, Cat# AF1924, RRID: AB 355060 |
| mouse anti-αSMA (smooth muscle actin) | 1: 500 | DAKO,Cat# M0851, RRID: AB 2223500 |
| mouse anti-MHC (myosin heavy chain) | 1: 1,000 | R&D Systems, Cat# MAB4470, RRID: AB 1293549 |
| mouse anti-TITIN | 1: 1,000 | DSHB, Cat# 9 D10, RRID: AB 528491 |
| rabbit anti-desmin | 1: 1,000 | Abeam, Cat# ab15200, RRID: AB 301744 |
| mouse anti-PAX7 | 1: 1,000 | DSHB, Cat# pax7, RRID: AB 528428 |
| rabbit anti-MYOD | 1: 1,000 | Proteintech, Cat# 18943-1-AP, RRID: AB 10603467 |
| rabbit anti-Ki67 | 1: 1,000 | Abeam, Cat# ab15580, RRID: AB 443209 |
| mouse anti-α-actinin | 1: 1,000 | Sigma-Aldrich, Cat# A7811, RRID: AB 476766 |
| mouse anti-myogenin | 1: 1,000 | Santa Cruz Biotechnology, Cat# sc-12732, RRID: AB 627980 |
| mouse anti-αA-crystallin | 1: 1,000 | Santa Cruz Biotechnology, Cat# sc-28306, RRID: AB 627304 |
| mouse anti-MAP2 | 1: 1,000 | Millipore, Cat# MAB3418, RRID: AB 94856 |
| rabbit anti-TBR1 | 1: 1,000 | Abeam, Cat# ab31940, RRID: AB 2200219 |
| | | |
| goat anti-rabbit IgG Alexa Fluor 488 | 1: 1,000 | Thermo Fisher Scientific Cat# A-11034, RRID: AB 2576217 |
| goat anti-mouse IgG Alexa Fluor 546 | 1: 1,000 | Thermo Fisher Scientific Cat# A-11030, RRID: AB 2534089 |
| goat anti-mouse IgM Alexa Fluor 546 | | Thermo Fisher Scientific Cat# A-21045, RRID: AB 2535714 |
| goat anti-mouse IgM Alexa Fluor 488 | 1: 1,000 | Thermo Fisher Scientific Cat# A32723, RRID: AB 2633275 |
| donkey anti-goat IgG Alexa Fluor 488 | 1: 1,000 | Thermo Fisher Scientific Cat# A-11055, RRID: AB 2534102 |
| goat anti-mouse IgM Alexa Fluor 488 | 1: 1,000 | Molecular Probes, Cat# A-21042, RRID: AB 141357 |
| chicken anti-mouse IgG Alexa Fluor 647 | 1: 1,000 | Thermo Fisher Scientific Cat# A-21463, RRID: AB 2535869 |
| chicken anti-mouse IgG Alexa Fluor 594 | 1: 1,000 | Molecular Probes, Cat# A-21201, RRID: AB_141630 |
| chicken anti-rabbit IgG Alexa Fluor 594 | 1: 1,000 | Molecular Probes, Cat# A-21442, RRID: AB_141840 |

An embryoid formation assay was performed to differentiate iPS cells into three germ layers (Suga, M. et al., Stem Cell Res. 36: 101406 (2019)). Embryoid bodies were formed by culturing iPS cells (9000 cells per embryoid body) dissociated with 0.5×TrypLE Select in a DMEM/F12 medium supplemented with 20% KSR, 2 mM L-glutamine, 0.1 mM NEAA, 0.1 mM 2-mercaptoethanol (all from Gibco (Thermo Fisher Scientific)), and 10 µM Y-27632 (NACALAI TESQUE) for 11 days using low adhesive surface V-bottom 96-well plates. They were then cultured for 7 days,in_DMEM supplemented with 10% FBS (Gibco (Thermo Fisher Scientific)) on tissue culture plates coated with Matrigel (BD Biosciences). The ability to differentiate into mesoderm was tested based on whether smooth muscle cells expressing smooth muscle actin differentiated. The ability to differentiate into endoderm was tested based on whether definitive endoderm cells expressing SOX17 differentiated. The ability to differentiate into ectoderm was tested based on whether motor neurons expressing βIII-tubulin differentiated.

Summary of cell types of iPS cell-derived differentiated cells used to examine the effect of ASO treatment on the formation of nuclear RNA aggregates, methods for culturing differentiated cells, methods for inducing differentiated cells from iPS cells, and methods for ASO treatment is shown in the following Table 6. Specific details are shown in the Examples using the respective differentiated cells.

**[Table 6]**

| evaluated cell | culture method | induction method | main reference relating to induction method | ASO treatment method |
|---|---|---|---|---|
| skeletal myocyte | 2D | direct transdifferentiation | Kokubu et al., 2019 DOI: 10.1002/sctm.18-0280 | lipofection 10nM |
| skeletal muscle organoid | 3D | organoid | Faustino Martins et al. 2020 DOI: 10.1016/j.stem.2019.12.007 | cultured for 7 days in 500 nM ASO-containing medium |
| nerve cell | 2D | direct transdifferentiation | Imamura et al., 2016 DOI: 10.1038/srep34904 | lipofection 10nM |
| lens epithelial cell | 2D | induced differentiation | Fu et al., 2017 DOI: 10.1167/iovs.16-2050 | lipofection 10nM |
| skin organoid | 3D | organoid | Lee et al., 2020 DOI: 10.1038/s41586-020-2352-3 | cultured for 7 days in 500 nM ASO-containing medium |

The bibliographic information of the references listed in Table 6 is as follows.
Kokubu, Y. et al. (2019) Stem Cells Transl Med, DOI: 10.1002/sctm.18-0280
Faustino Martins, J.M. et al. (2020) Cell stem Cell, 26: 172-186 e176.
Imamura, K. et al. (2016) Sci Rep., 6:34904.
Fu, Q. et al. (2017) Invest Ophthalmol Vis Sci. 58: 517-527. Lee, J. et al. (2020) Nature, 582: 399-404.

In order to detect nuclear RNA aggregates, fluorescence in situ hybridization (hereinafter to be referred to as "FISH") using Cy3 labeled (CAG)₆-CA DNA/LNA probe was performed. Cells were cultured on clear-bottomed microplates (CellCarrier-96 Ultra, PerkinElmer), fixed with 4% paraformaldehyde for 30 min at room temperature, membrane permeabilized with 70% ethanol at -30°C for at least 2 hr, rehydrated with a 1×SSC solution prepared with diethyl dicarbonate (DEPC) water, and incubated in a hybridization pre-treatment buffer [2×SSC and 50% formamide prepared with DEPC water] at 55°C for 2 hr. Thereafter, the cells were incubated in a hybridization buffer [2×SSC, 50% formamide, 10% dextrin sulfate and 0.1 ng/µL Cy3 labeled (CAG)₆-CA DNA/LNA probe (GeneDesign, Inc., Osaka)] overnight at 55°C (de Mezer, M. et al., Nucleic Acids Res, 39:3852(2011)). The cells were washed twice with the aforementioned hybridization pre-treatment buffer at 55°C for 20 min, and washed twice at room temperature for 5 min with 1×SSC solution prepared with DEPC water. The cells were further incubated with 1×SSC prepared in DEPC water at 37°C for 60 min and washed twice with 1×SSC prepared in DEPC water. The cells were stained with 1 µg/mL 4',6-diamidino-2-phenylindole (DAPI, Invitrogen, Thermo Fisher Scientific) in PBS at room temperature for 10 min and washed with PBS. Imaging was analyzed with the Opera Phenix (trademark) High Content Screening System (PerkinElmer) using a 40x water immersion objective lens.

### (1.2) results

iPS cells derived from 3 DM1 patients of the experimental group and 3 healthy subjects of the control group were respectively designated DM-1 to -3 and HC-1 to -3. Mycoplasma contamination was not detected in cell culture. Table 7 shows the starting cells of DM-1 to -3 and HC-1 to -3, gender and age of the donors, the repeat number of the repeat number sequence of the CTG sequence analyzed by Southern blot method, and the induction vector of pluripotent stem cells. In the starting cells of Table 7, PBMC means peripheral blood mononuclear cells.

**[Table 7]**

| | iPS cells derived from healthy subject of control group | | | iPS cells derived from DM1 patient | | |
|---|---|---|---|---|---|---|
| | HC-1 | HC-2 | HC-3 | DM-1 | DM-2 | DM-3 |
| clone ID | HPS0076 | HPS3835 | HPS3838 | HPS1033 | HPS1051 | HPS1052 |
| starting cell | skin fibroblast | PBMC | PBMC | PBMC | PBMC | PBMC |
| gender | female | male | female | male | female | male |
| age | 36 | 47 | 46 | 38 | 33 | 38 |
| repeat number | N/A | N/A | N/A | 1000-1150 | 150 | 2300-2850 |
| induction vector | Episomal | Episomal | Episomal | Episomal | Episomal | Episomal |

Fig. 1A is a panel of microscope photographs showing the expression of undifferentiated pluripotent stem cell markers of DM-1 to -3 and HC-1 to -3. The top row shows phase contrast microscope photographs of monolayer culture of the undifferentiated cells of respective iPS cells, and the second row shows fluorescence microscope photographs of monolayer culture of the undifferentiated cells of respective iPS cells, that were stained with an antibody against NANOG and then subjected to counterstaining of the cell nuclei with DAPI. The third row shows fluorescence microscope photographs of monolayer culture of the undifferentiated cells of respective iPS cells, that were stained with an antibody against OCT4 and then subjected to counterstaining of the cell nuclei with DAPI. The fourth row shows fluorescence microscope photographs of monolayer culture of the undifferentiated cells of respective iPS cells, that were stained with an antibody against SSEA4 and then subjected to counterstaining of the cell nuclei with DAPI. The fifth row shows fluorescence microscope photographs of monolayer culture of the undifferentiated cells of respective iPS cells, that were stained with an antibody against TRA-1-60 and then subjected to counterstaining of the cell nuclei with DAPI. The scale bar on the lower right of each microscope photograph is 200 µm.

As shown in Fig. 1A, DM-1 to -3 and HC-1 to -3 all showed human undifferentiated embryonic stem cell-like morphology in which cells adhere tightly to each other and the borders of individual cells cannot be distinguished under a phase-contrast microscope. In addition, DM-1 to -3 and HC-1 to -3 all expressed NANOG, OCT4, SSEA4, and TRA-1-60, which are markers of human iPS undifferentiated cells, and exhibited characteristics of human iPS undifferentiated cells.

Fig. 1B is a panel of microscope photographs showing the expression of three germ layer differentiation markers in cells differentiated from DM-1 to -3 and HC-1 to -3. The first row shows fluorescence microscope photographs of cells differentiated into mesoderm from respective iPS cells, that were stained with an antibody against α-smooth muscle actin (SMA) and then subjected to counterstaining of the cell nuclei with DAPI. The second row shows fluorescence microscope photographs of cells differentiated into endoderm from respective iPS cells, that were stained with an antibody against SOX17 and then subjected to counterstaining of the cell nuclei with DAPI. The third row shows fluorescence microscope photographs of cells differentiated into ectoderm from respective iPS cells, that were stained with an antibody against βIII-tubulin and then subjected to counterstaining of the cell nuclei with DAPI. The scale bar on the lower right of each fluorescence microscope photograph is 50 µm.

As shown in Fig. 1B, DM-1 to -3 and HC-1 to -3 all showed the ability to differentiate into all of mesodermal cells (α-smooth muscle actin), entodermal cells (SOX17), and ectodermal cells (βIII-tubulin). Thus, iPS 1 to 3 and HC-1 to -3 were all proved to be human iPS undifferentiated cells with pluripotency.

Fig. 1C shows capillary electrophoresis of reaction products of TP-PCR of DM-1 to -3 and HC-1 to -3. As shown in Fig. 1C, the repeat numbers of the CTG repeat sequences of the DMPK genes of DM-1 to -3 and HC-1 to -3 are consistent with the repeat numbers of genomic DNA of the donor analyzed by the Southern blot method shown in Table 7.

Fig. 2 is a panel of fluorescence microscope photographs showing the results of fluorescence in situ hybridization (hereinafter to be referred to as "FISH") of iPS cells (DM-3) derived from DM1 patient, using Cy3 labeled (CAG)₆-CA DNA/LNA probe. The upper left and the lower left show the results of FISH without RNase A treatment, and the upper right and the lower right show the results of FISH after RNase A treatment. The upper left and the upper right are fluorescence microscope photographs with counterstaining of the cell nuclei with DAPI after FISH, and the lower left and the lower right are fluorescence microscope photographs with FISH alone and without DAPI staining.

According to Fig. 2, the bright spots observed when FISH was performed without RNase A treatment disappeared when FISH was performed after RNase A treatment. The experiment results indicate that the aforementioned bright spots were signals of CUG-RNA aggregates (CUG-RNA foci) hybridized with the probe.

Fig. 3A is a panel of fluorescence microscope photographs showing the results of FISH of respective undifferentiated cells of DM-1 to -3 and HC-1 to -3, using Cy3 labeled (CAG)₆-CA DNA/LNA probe. The upper right corner of each photograph is a high magnification fluorescence microscope photograph of one nucleus. The scale bar on the lower right of each fluorescence microscope photograph is 10 µm.

Based on the FISH results in Fig. 3A, image analysis of RNA aggregates was performed using Opera Phenix (trademark) High Content Screening System (PerkinElmer).

Fig. 3B, left, is a bar graph showing the average number of RNA aggregates per cell nucleus calculated from the FISH results in Fig. 3A. Fig. 3B, right, is a bar graph showing the percentage of RNA aggregate-positive cell nuclei detected by DAPI staining in total cell nuclei, calculated from the FISH results in Fig. 3A. "**" indicates that the numerical values of DM-1 to - 3 are significantly different from the numerical values of HC-1 to -3 (p<0.01).

As a result of the image analysis shown in Fig. 3B, it was clarified that the number of RNA aggregates per cell nucleus and the number of cells having CUG-RNA aggregates significantly increased in the iPS cells derived from DM1 patient as compared with the iPS cells derived from healthy subject. In addition, among the iPS cells derived from DM1 patient, DM-1 (more than 1000 times) and DM-3 (more than 2300 times), which have a large number of repeats of the CTG sequence as shown in Table 7, showed the number of RNA aggregates as high as 3.32±0.23 (mean±standard deviation) in DM-1 and 1.87±0.23 in DM-3, as shown in Fig. 3B. In contrast, DM-2, which has a low number of repeats of the CTG sequence (more than 150 times), showed a significantly lower number of RNA aggregates of 0.078±0.009 (p<0.01).

From the above results, it was found that the DM1 patient-derived iPS prepared this time has the basic characteristics of pluripotent stem cells and reproduces the cell phenotype of myotonic dystrophy type 1 patients.

### Example 2 Evaluation of ASO using iPS cells derived from DM1 patient (1) undifferentiated cell

### (2.1) material and method

Fig. 4A is a schematic diagram showing the relative positional relationship on DMPK-pre mRNA of the complementary sequences of ASOs of the Examples of the present application, and also showing the structural types of ASOs. ASO-1 to -3 are complementary to sequences in the noncoding region of exon 15 on DMPK-pre mRNA, ASO-4 is complementary to repeated sequence of CUG sequence in the noncoding region of exon 15 on DMPK-mRNA, and ASO-5 is complementary to sequence on the 3' end side of the repeated sequence in the noncoding region of exon 15 on pre DMPK-mRNA. ASO-4 is a mixmer consisting of ENA and 2'-OMe-nucleotide analog. Other ASO-1 to -3, and 5 are gapmers consisting of ENA in the wing region and unmodified deoxyribonucleotides in the gap region (hereinafter to be referred to as "ENA/DNA gapmer"). ASO-C used as a negative control in the Examples of the present specification is an ENA/DNA gapmer that targets transcription products of genes completely different from DMPK.

Fig. 5A is a schematic diagram showing the relative positional relationship on DMPK-pre mRNA of the complementary sequences of ASO-2 and -4, and control ASO-A and -B respectively corresponding thereto of the Examples of the present application, and also showing the structural types of ASOs. ASO-2 and ASO-A are complementary to the same sequence in the coding region of exon 15 on DMPK-mRNA, and ASO-4 and ASO-B are complementary to the same sequence of repeated sequence of CUG sequence in the noncoding region of exon 15 on DMPK-mRNA. ASO-2 is an ENA/DNA gapmer, whereas ASO-A is a gapmer consisting of MOE-nucleotide analog in the wing region and unmodified deoxyribonucleotides in the gap region (hereinafter to be referred to as "MOE/DNA gapmer"). ASO-4 is a mixmer consisting of ENA and 2'-OMe-nucleotide analog, and ASO-B consists of 2'-OMe-nucleotide analogues at all nucleotides (RNase H inactive nucleotide).

The base sequences and structural features of ASO-1 to -5 and ASO-A to -C are shown in Tables 2 and 3 in the Detailed Description of the Invention of the present specification. The base sequences of ASO-1 to -4 and -5 are listed in SEQ ID NOs: 11 to 14 and 3, respectively, in the Sequence Listing attached to the specification. The base sequence of ASO-A is listed in SEQ ID NO: 10. ASO-A and -B have the same base sequence as ASO-1 and -5, respectively. ENA was obtained from KNC Laboratories Co., Ltd. (Kobe) and oligonucleotides were synthesized. Administration of ASO to cultured iPS cells was performed according to the manufacturer's instructions by using ASO at a final concentration of 5 or 10 nM together with DharmaFECT1 (Horizon Discovery, Cambridge, UK). FISH using Cy3 labeled (CAG)₆-CA DNA/LNA probe was performed 48 hr after administration of ASO to the cultured iPS cells.

### (2.2) results

Fig. 4B is a fluorescence microscope photograph of undifferentiated iPS cells (DM-1) derived from DM1 patient and administered with either ASO-C or ASO-1 to -5, followed by FISH using Cy3 labeled (CAG)₆-CA DNA/LNA probe and DAPI counterstaining 48 hr later. The scale bar on the lower right of each fluorescence microscope photograph is 10 µm.

Fig. 4C is a bar graph showing the average number of RNA aggregates per cell nucleus calculated from the FISH results of • undifferentiated iPS cells (DM-1) derived from DM1 patient and administered with 5 nM or 10 nM of either ASO-C or ASO-1 to -5.

Fig. 4D is a bar graph showing the percentage of RNA aggregate-positive cell nuclei detected by DAPI staining in total cell nuclei, calculated from the FISH results of undifferentiated iPS cells (DM-1) derived from DM1 patient and administered with 5 nM or 10 nM of either ASO-C or ASO-1 to -5.

As shown in Figs. 4B to 4D, all of ASO-1 to -5 significantly reduced RNA aggregates in undifferentiated iPS cells derived from DM1 patient. Among ASO-1 to -3 and -5 of ENA/DNA gapmer, ASO-5 suppressed the formation of RNA aggregates most strongly (Figs. 4C and 4D). When iPS cells were treated with mixmer ASO-4, which consists of ENA and 2'-OMe-nucleotide analog targeting CUG repeat sequence, RNA aggregates disappeared completely (Figs. 4B to 4D).

Fig. 4E is a bar graph showing the state of proliferation and survival of undifferentiated iPS cells (DM-1) derived from DM1 patient and administered with 5 nM or 10 nM of either ASO-C or ASO-1 to -5. The vertical axis shows the ratio of the number of cells with the number of ASO-C cells as 1.0.

As shown in Fig. 4E, ASO-1, -4 and -5 do not affect the proliferation and survival of undifferentiated iPS cells at either 5 nM or 10 nM, whereas administration of ASO-2 and -3 caused a decrease in the cell number even at a low dose.

As shown in Fig. 5A, ASO-2 and ASO-A target the same sequence on DMPK-mRNA. However, they are different in that ASO-2 is an ENA/DNA gapmer, whereas ASO-A is an MOE/DNA gapmer. ASO-A was reported under the code name ISIS 445569 (Non Patent Literature 5 (Wheeler, T.M. et al., Nature, 488, 111(2012))). Similarly, ASO-4 and ASO-B target the same sequence on DMPK-mRNA. However, they are different in that ASO-4 is a mixmer consisting of ENA and 2'-OMe-nucleotide analog, whereas ASO-B is a mixmer consisting of all nucleotides being 2'-OMe-nucleotide analogs. ASO-B was reported under the code name PS58 (Non Patent Literature 7 (Mulders, S.A. et al., Proc Natl Acad Sci USA, 106:13915(2009))). Therefore, ASO-A and -B were prepared this time and used as positive controls for ASO-2 and -4, respectively.

Fig. 5B is a fluorescence microscope photograph of undifferentiated iPS cells (DM-1) derived from DM1 patient and administered with ASO-2 or ASO-A, followed by FISH using Cy3 labeled (CAG)₆-CA DNA/LNA probe and DAPI counterstaining. The scale bar on the lower right of each fluorescence microscope photograph is 10 µm.

Fig. 5C, left, is a bar graph showing the average number of RNA aggregates per cell nucleus calculated from the FISH results of undifferentiated iPS cells (DM-1) derived from DM1 patient and administered with 5 nM or 10 nM of ASO-2, ASO-A, or ASO-C. Fig. 5C, middle, is a bar graph showing the percentage of RNA aggregate-positive cell nuclei detected by DAPI staining in total cell nuclei, calculated from the FISH results of undifferentiated iPS cells (DM-1) derived from DM1 patient and administered with 5 nM or 10 nM of ASO-2, ASO-A, or ASO-C. Fig. 5C, right, is a bar graph showing the state of proliferation and survival of undifferentiated iPS cells (DM-1) derived from DM1 patient and administered with 5 nM or 10 nM of ASO-2, ASO-A, or ASO-C. The vertical axis shows the ratio of the number of cells with the number of ASO-C cells as 1.0.

Fig. 5D is a fluorescence microscope photograph of undifferentiated iPS cells (DM-1) derived from DM1 patient and administered with ASO-4 or ASO-B, followed by FISH using Cy3 labeled (CAG)₆-CA DNA/LNA probe and DAPI counterstaining. The scale bar on the lower right of each fluorescence microscope photograph is 10 µm.

Fig. 5E, left, is a bar graph showing the average number of RNA aggregates per cell nucleus calculated from the FISH results of undifferentiated iPS cells (DM-1) derived from DM1 patient and administered with 5 nM or 10 nM of ASO-4, ASO-B, or ASO-C. Fig. 5E, middle, is a bar graph showing the percentage of RNA aggregate-positive cell nuclei in total cell nuclei, calculated from the FISH results of undifferentiated iPS cells (DM-1) derived from DM1 patient and administered with 5 nM or 10 nM of ASO-4, ASO-B, or ASO-C. Fig. 5E, right, is a bar graph showing the state of proliferation and survival of undifferentiated iPS cells (DM-1) derived from DM1 patient and administered with 5 nM or 10 nM of ASO-4, ASO-B, or ASO-C. The vertical axis shows the ratio of the number of cells with the number of ASO-C cells as 1.0.

As shown in Fig. 5C, both at 5 nM and 10 nM, ASO-A reduced RNA aggregates without reducing the number of undifferentiated iPS cells, whereas ASO-2 reduced both RNA aggregates and the number of undifferentiated iPS cells. Therefore, the effects of ASO-2 are considered to be due to cytotoxicity.

In contrast, as shown in Fig. 5E, both at 5 nM and 10 nM, both ASO-4 and ASO-B reduced RNA aggregates without reducing the number of undifferentiated iPS cells. Therefore, the effects of ASO-4 and ASO-B are not due to cytotoxicity. Moreover, ASO-4 almost completely eliminates RNA aggregates, and thus can be said to afford a remarkably superior RNA aggregate inhibitory effect compared to ASO-B in which more than half of the RNA aggregates remain. The difference between ASO-B and ASO-4 is that ASO-4 is a mixmer consisting of ENA and 2'-OMe-nucleotide analog, whereas ASO-B consists of 2'-OMe-nucleotide analogues at all nucleotides in which all bonds between nucleotides are phosphorothioate. Thus, it is considered that the mixmer structure containing at least two types of RNase H inactive nucleotide analogs and/or at least two types of internucleotide linkage achieved the remarkably superior RNA aggregate suppressive effect of ASO-4.

### Example 3 Differentiation of myocyte from iPS cells derived from myotonic dystrophy patient

### (3.1) material and method

A method for differentiating muscle cells from undifferentiated iPS cells established in the Examples of the present invention is as follows. The Tet-On inducible MyoD expression system (Non Patent Literature 22, Shoji, E. et al., Sci Rep, 5, 12831(2015)) and the puromycin resistance marker were incorporated into the piggyBac vector KW110_PB_TA_ERN (Addgene, Watertown, Massachusetts, USA). The prepared vector was co-transfected into undifferentiated cells of iPS cells (DM-1 to -3) derived from DM1 patient and iPS cells (HC-1 to - 3) derived from healthy subject, using lipofectamine LTX (Thermo Fisher Scientific K.K.) and according to the manufacturer's instructions, along with pCyL43 vector encoding transposase. iPS cell clone was selected using puromycin (NACALAI TESQUE, INC.). iPS cells were seeded in StemFit medium supplemented with 10 µM Rock inhibitor Y27632 (NACALAI TESQUE, INC.) on Matrigel-coated cell culture vessels, and cultured for 2 days, after which the medium was replaced with α-MEM (NACALAI TESQUE, INC.) supplemented with 5% KnockOut (trademark) Serum Replacement (KSR, Thermo Fisher Scientific K.K.), 200 µM 2-mercaptoethanol (Thermo Fisher Scientific K.K.), 100 U/mL penicillin/streptomycin (Thermo Fisher Scientific K.K.), and 1 µg/mL doxycycline (Clontech, Takara Bio Inc., Shiga). Doxycycline was removed on day 8. The medium was changed every other day.

Fig. 6A is a conceptual diagram showing procedures for an experimental system for differentiation of undifferentiated cells of iPS cells (DM-1 to -3) derived from DM1 patient and iPS cells (HC-1 to -3) derived from healthy subject into myocytes, using a tetracycline-inducible MyoD expression system. "StemFit" shows the period of culture in the medium for undifferentiated iPS cells, "KSR/α-MEM" shows the period of culture in a medium for differentiated myocytes, and "KSR/α-MEM+Dox" shows the period during which cells were cultured in a medium for myocytes supplemented with doxycycline as a differentiation-inducing factor. Undifferentiated iPS cells were transfected on day 0 with a tetracycline-inducible MyoD expression system, the medium was changed to a myocyte medium supplemented with a differentiation-inducing factor on day 2 to initiate differentiation induction, the differentiation induction was terminated on day 8 by changing to a myocyte medium containing no differentiation-inducing factor, and analysis was performed on day 12.

Myocytes differentiated from undifferentiated cells of DM-1 to -3 and HC-1 to -3 cells were analyzed using the immunocytechemical method and nuclear RNA aggregate detection method described in Example 1.

### (3.2) results

Fig. 6B is a panel of fluorescence microscope photographs showing the expression of muscle differentiation marker in cells induced to differentiate from the undifferentiated cells of iPS cells (HC-1 to -3) derived from healthy subject and iPS cells (DM-1 to -3) derived from DM1 patient according to the procedures shown in Fig. 6A. The upper panel shows fluorescence microscope photographs of cells stained with an antibody against myosin heavy chain (MHC) and then subjected to counterstaining of the cell nuclei with DAPI. The middle panel shows fluorescence microscope photographs of cells stained with an antibody against α-actinin and then subjected to counterstaining of the cell nuclei with DAPI. The lower panel shows fluorescence microscope photographs of cells stained with an antibody against myogenin (MyoG) and then subjected to counterstaining of the cell nuclei with DAPI.

As shown in Fig. 6B, both HC-1 to -3 and DM-1 to -3 could be induced to differentiate into skeletal muscle cells expression myosin heavy chain (MHC), α-actinin, and myogenin, by using a tetracycline-inducible MyoD expression system.

Fig. 6C is a bar graph showing the percentage of MHC-expressing cell nucleus (MHC/DAPI) in the myocytes differentiated from the undifferentiated cells of the cells of HC-1 to -3 and DM-1 to -3 according to the procedures shown in Fig. 6A. The "n.s." shows that no significant difference was found between HC-1 to -3 cells and DM-1 to -3 cells.

Fig. 6D is a bar graph showing the percentage of cell nuclei (α-actinin/DAPI) that express muscle differentiation marker α-actinin among the DAPI-staining positive cell nuclei of myocytes differentiated from the undifferentiated cells of the cells of HC-1 to -3 and DM-1 to -3 according to the procedures shown in Fig. 6A. The "n.s." shows that no significant difference was found between HC-1 to -3 cells and DM-1 to -3 cells.

Fig. 6E is a bar graph showing the percentage of cell nuclei (myogenin/DAPI) that express muscle differentiation marker myogenin among the DAPI-staining positive cell nuclei of myocytes induced to differentiate from the undifferentiated cells of HC-1 to -3 and DM-1 to -3 according to the procedures shown in Fig. 6A. The "n.s." shows that no significant difference was found between HC-1 to -3 cells and DM-1 to -3 cells.

Fig. 6F is a panel of fluorescence microscope photographs showing the results of FISH using Cy3 labeled (CAG)₆-CA DNA/LNA probe of the myocytes differentiated from the cells of HC-1 to -3 and DM-1 to -3 according to the procedures shown in Fig. 6A. The scale bar on the lower right of each fluorescence microscope photograph is 10 µm.

Fig. 6G, left, is a bar graph showing the average number of RNA aggregates per cell nucleus calculated from the FISH results of respective myocytes differentiated from the cells of HC-1 to -3 and DM-1 to -3 according to the procedures shown in Fig. 6A. Fig. 6G, right, is a bar graph showing the percentage of RNA aggregate-positive cell nuclei detected by DAPI staining in total cell nuclei, calculated from the FISH results of respective myocytes differentiated from the cells of HC-1 to -3 and DM-1 to -3 according to the procedures shown in Fig. 6A. "**" indicates that the numerical values of DM-1 to - 3 are significantly different from the numerical values of HC-1 to -3 (p<0.01).

As shown in Figs. 6B to 6D, not less than 70% of myocytes derived from HC-1 to -3 and DM-1 to -3 cells expressed myosin heavy chain (MHC) and α-actinin. As shown in Fig. 6E, in myocytes derived from HC-1 and DM-1 cells, 60-70% of DAPI-staining positive cell nuclei expressed myogenin. However, in myocytes derived from HC-2 and -3 cells and DM-2 and -3 cells, only 20-40% of DAPI-staining positive cell nuclei expressed myogenin.

### Example 4 Evaluation of ASO using iPS cells derived from DM1 patient(2) myocyte

### (4.1) material and method

The myocytes derived from HC-1 to -3 and DM-1 to -3 explained in Example 3 were analyzed using the method for detecting nuclear RNA aggregates described in Example 1 and the method for administering ASO to cultured iPS cells described in Example 2.

### (4.2) results

Fig. 6F is a panel of fluorescence microscope photographs showing the results of FISH using Cy3 labeled (CAG)₆-CA DNA/LNA probe of the myocytes differentiated from the cells of HC-1 to -3 and DM-1 to -3 according to the procedures shown in Fig. 6A. The scale bar on the lower right of each fluorescence microscope photograph is 10 µm.

Fig. 6G, left, is a bar graph showing the average number of RNA aggregates per cell nucleus calculated from the FISH results of respective myocytes differentiated from the cells of HC-1 to -3 and DM-1 to -3 according to the procedures shown in Fig. 6A. Fig. 6G, right, is a bar graph showing the percentage of RNA aggregate-positive cell nuclei detected by DAPI staining in total cell nuclei, calculated from the FISH results of respective myocytes differentiated from the cells of HC-1 to -3 and DM-1 to -3 according to the procedures shown in Fig. 6A. "**" indicates that the numerical values of DM-1 to - 3 are significantly different from the numerical values of HC-1 to -3 (p<0.01) .

As shown in Fig. 6F and 6G, RNA aggregates were detected in the myocytes derived from iPS cells derived from DM1 patient (DM-1 to -3). By comparison of the graphs of Fig. 3B and Fig. 6G, there is no significant difference in the percentage of RNA aggregate-positive cell nuclei in total cell nuclei between undifferentiated DM-1 to -3 cells and myocytes derived from DM-1 to -3. However, a very large difference is observed in the average number of RNA aggregates per cell nucleus between undifferentiated DM-1 and -3 cells (2 - 3.5) and myocytes derived from DM-1 and -3 (11 - 16). Almost no RNA aggregates were detected in myocytes derived from DM-2.

Fig. 7A is a panel of fluorescence microscope photographs showing FISH using Cy3 labeled (CAG)₆-CA DNA/LNA probe and DAPI counterstaining after administration of 10 nM of ASO-C or ASO-4 to myocytes derived from DM-1 to -3 by lipofection.

Fig. 7B is a bar graph showing the average number of RNA aggregates per cell nucleus calculated from the FISH results of myocytes derived from DM-1 to -3 cells administered with 10 nM of ASO-C or ASO-4.

Fig. 7C is a bar graph showing the percentage of RNA aggregate-positive cell nuclei (ASO-C-administered myocytes as 100%) detected by DAPI staining in total cell nuclei, calculated from the FISH results of myocytes derived from DM-1 to -3 cells administered with 10 nM of ASO-C or ASO-4.

Fig. 7D is a bar graph showing the state of proliferation and survival of myocytes derived from DM-1 to -3 cells administered with 10 nM of ASO-C or ASO-4. In any bar graph, the vertical axis shows the ratio of the number of cells with the number of ASO-C cells as 1.0.

As shown in Fig. 7A - 7D, as compared with ASO-C, administration of ASO-4 remarkably decreased the average number of RNA aggregates per cell nucleus in the myocytes derived from DM-1 and -3 cells. The administration of ASO-4 hardly changed the average number of RNA aggregates per cell nucleus in myocytes derived from DM-2 cells. This may be related to the fact that DM-2 cells were derived from patients with a low repeat number of the CTG sequence of DMPK gene. As shown in Table 7 and Fig. 1C, donors of DM-2 cells have at most 150 repeats of the CTG sequence which is very small compared to the repeat numbers of the CTG sequence of 1000 - 1150 and 2300 - 2850 in the donors of DM-1 and -3 cells, respectively. Therefore, it is considered that the effect of reducing RNA aggregates by ASO-4 administration is less apparent in DM-2 cells than in DM-1 and DM-3 cells.

From the above results, it was concluded that ASO-4 can decrease RNA aggregates in the skeletal muscle cells derived from the iPS cells derived from DM1 patient.

### Example 5 Differentiation of neuromuscular organoid from iPS cells derived from myotonic dystrophy patient

### (5.1) material and method

Neuromuscular organoids were produced by adding some modifications to the protocol published by Faustino Martins, J.M. et al. (Cellstem Cell, 26: 172-186 e176. (2020)). As shown schematically in the schematic diagram of Fig. 8A, 70% confluent iPS cells were dissociated into single cells and seeded in Neurobasal (NB) medium supplemented with 10 µM Y27632, 3 µM CHIR99021 (Tocris Bioscience), and 40 ng/mL FGF-2 (Wako) at a density of 75,000 cells/cm² on Matrigel-coated culture vessels. NB was a 1:1 mixture of Advanced Dulbecco's Modified Eagle medium F12 (Gibco) supplemented with 1×N2(Gibco), and Neurobasal medium (Gibco) supplemented with 1×B27(Gibco), 2 mM L-glutamine (Gibco), 75 µg/mL BSA fraction V (Sigma), and 0.1 mM 2-mercaptoethanol (Gibco). The next day, Y27632 was removed and cells were differentiated into neural mesoderm progenitor cells (NMPs) for 2 days. On day 0 of organoid formation, mNMPs were dissociated with Accumax (Innovative Cell Technologies), and 4,500 to 7,200 cells suspended in NB medium (100 µL) supplemented with 50 µM Y27632, 10 ng/mL FGF-2, 2 ng/mL IGF, and 2 ng/mL HGF (Peprotech) per well were seeded in PrimeSurfce 96-well plates. On day 2, half of the culture medium was replaced with NB medium supplemented with 2 ng/ml IGF and 2 ng/ml HGF. Organoids were maintained in NB medium from day 4. On day 10, organoids were transferred to 60 mm culture dishes, and transferred to 100 mm culture dishes on day 30. Organoids were spin cultured at 75 rpm and the medium was changed to a fresh NB medium twice a week. For ASO treatment of organoids, organoids were spin-cultured in NB medium containing 500 nM ASO.

The immunohistochemical examination of organoids was performed as follows.

Organoids were fixed with 4% paraformaldehyde at room temperature for 15 min, washed three times in PBS for 10 min, and immersed overnight in a 30% sucrose solution to allow formation of sediment. Organoids were embedded in OCT Compound (Sakura Finetech Japan Co., Ltd., Tokyo) and rapidly frozen in liquid nitrogen. Frozen organoids were cryosectioned at -18°C to -20°C using cryostat (CM1850, Leica Microsystems, Wetzlar, Germany) into 12 µm thick frozen sections. For immunohistochemistry test, the sections were permeabilized in 0.5% Triton-X100/PBS (0.5% PBST) for 30 min at room temperature and incubated in Blocking One Histo (NACALAI TESQUE) at room temperature for 2 hr. Sections were incubated with primary antibody in a blocking solution overnight at 4°C. Samples were then washed 4 times with 0.1% PBST for 15 min each time and incubated with the secondary antibody at room temperature for 2 hr in shading. Thereafter, the samples were washed 4 times with 0.1% PBST for 15 min each time, and mounted with ProLong (trademark) Gold Antifade Mountant (Thermo Fisher Scientific). The data was acquired using a confocal laser microscope (Nikon A1, Nikon, Tokyo). For image analysis, NIS-Element AR Analysis (version 5.11.0.1, Nikon) was used.

### (5.2) results

In order to verify whether the ASO according to the present invention reduces or eliminates RNA aggregates not only in myocytes but also in muscle-differentiating progenitor cells and skeletal muscle fibers, neuromuscular organoids (NMOs) were three-dimensionally cultured for 50 days according to the method reported by Faustino Martins, J.M. et al. The three-dimensional aggregates are formed from neuromuscular progenitor cells (NMPs) derived from neuromuscles derived from iPS cells, and are destined to differentiate neuroectodermal and mesodermal lineage cells. As shown in Fig. 8B, the three-dimensional aggregates grew into organoids containing neural and mesodermal regions by day 5, and on day 50, NMOs were observed to have a neural compartment with many TUJ1-expressing neurons and a skeletal muscle compartment with many MHC-expressing cells. As shown on the left side of the panel of Fig. 8B, immunofluorescence analysis of the muscle region of NMOs on day 50 revealed the expression of sarcomere protein TITIN in skeletal muscle fibers.

As shown in the upper row of Fig. 8C, muscle progenitor cells/satellite cells expressing PAX7 were observed along myocytes and myotubes expressing desmin in the skeletal muscle compartment of the organoid. As shown in the lower row of Fig. 8C, RNA aggregates were detected in both PAX7-expressing cells and desmin-expressing cells, in the neuromuscular organoid derived from the iPS cells of DM1 patient.

### Example 6 Evaluation of ASO using iPS cells derived from DM1 patient (3) neuromuscular organoid

### (6.1) material and method

Differentiation into neuromuscular organoids from iPS cells derived from healthy subject and myotonic dystrophy patient and immunohistochemical examination of the organoids were performed in the same manner as in Example 5. In order to detect nuclear RNA aggregates, FISH using Cy3 labeled (CAG)₆-CA DNA/LNA probe was performed in the same manner as in Example 1. For ASO treatment of organoids, organoids were spin-cultured for 7 days in an NB medium containing 500 nM ASO.

### (6.2) results

As shown in the lower row of Fig. 8C and Fig. 8D, when neuromuscular organoids derived from the iPS cells of DM1 patient were treated with 500 nM ASO-4M, the number of RNA aggregates was reduced in both desmin-expressing myocytes and PAX7-expressing muscle progenitor cells/satellite cells.

Also, from the upperrow of photographs in Fig. 8C, when compared with the neuromuscular organoids derived from the iPS cells of healthy subjects, it was observed that the number of muscle progenitor cells/satellite cells expressing PAX7 on day 50 of culture of neuromuscular organoids derived from the iPS cells of DM1 patient was reduced. Therefore, in the skeletal muscle compartment of NMOs, the ratio of the number of PAX7-expressing cells to the total number of cells (PAX7⁺/DAPI), and the ratio of the number of PAX7-expressing cells and the number of cells with muscle differentiation potency that express MYOD (PAX7⁺/MYOD⁺) was analyzed (Fig. 8 E and F). As a result, when compared with the neuromuscular organoids derived from healthy subject, the number of muscle progenitor cells/satellite cells expressing PAX7 was significantly reduced in neuromuscular organoids derived from DM1 patient. Conversely, the ratio of the number of cells with muscle differentiation potency that express MYOD to the total number of cells, and the ratio of the number of cells expressing Ki67 to the number of muscle progenitor cells/satellite cells expressing PAX7 did not change between DM1 patient and healthy subject. These results suggest that the satellite cells decreased in neuromuscular organoids of DM1 patient, but that ASO-4M treatment restored them to the same level as neuromuscular organoids of healthy subject.

From the results of this Example, DM1-specific nuclear RNA aggregate formation could be detected in both desmin-expressing myocytes and PAX7-expressing muscle progenitor cells/satellite cells. It was also clarified that cells expressing PAX7 were reduced in neuromuscular organoids of DM1 patient. It was suggested that the action of DMPK RNA, in which the repeat sequence of the CUG sequence is extended, possibly affects the entire process of skeletal muscle differentiation from satellite cell behavior to muscle fiber formation. Therefore, the experimental system of three-dimensional muscle organoid culture can provide a new aspect as an in vitro disease model.

### Example 7 Differentiation of lens epithelial cells and lens fiber cells from iPS cells derived from myotonic dystrophy patient

### (7.1) material and method

Lens epithelial cells and lens fiber cells were generated from undifferentiated iPS cells according to a reference, Fu, Q. et al. (Invest Ophthalmol Vis Sci. 58: 517-527 (2017)), listed in Table 6. First, about 80 colonies of about 20 iPS cells each were seeded and cultured in mTesR medium (Stemcell, Vancouver, Canada) in a Matrigel-coated culture dish. Four hours after plating, iPS cells were induced via 100 ng/ml of the BMP inhibitor noggin, and induced to differentiate into ectoderm/nerve ectoderm until epithelial-like cells first appeared on the periphery of the colonies on day 6 of culture. About 50 differentiated iPS cells were then mechanically dissociated together with the peripheral epithelial-like cells, and colonies of 30 to 50 differentiated iPS cells were selected and reseeded on matrigel-coated culture dish. The BMP signal was then reactivated via its agonists BMP4 and BMP7 (20 ng/ml). Simultaneously, fibroblast growth factor signaling was activated by bFGF (100 ng/ml). Cell clusters containing lens epithelial cells that were morphologically different from the surrounding cells and spread over the culture substrate were observed on day 11 of culture. On day 15 of culture, BMP4 and BMP7 were replaced with Wnt3a (20 ng/ml) to activate Wnt signal and differentiation of lens epithelial cells into lens fiber cells was induced.

The differentiation of lens epithelial cells and lens fiber cells was confirmed using αA-crystallin as a marker. Immunocytochemical detection of anti-αA-crystallin antibody was performed in the same manner as in Example 1. In addition, iPS cell-derived cultures containing cells differentiated into lens epithelial cells and lens fiber cells were analyzed using the detection method of nuclear RNA aggregates described in Example 1.

### (7.2) results

Fig. 9A shows phase contrast microscope photographs of lens epithelial cells and lens fiber cells derived from iPS cells of healthy subject and DM1 patient (HC and DM1, respectively). The scale bar in Fig. 9A is 100 µm. Fig. 9B shows fluorescence microscope photographs of lens epithelial cells and lens fiber cells (HC and DM1, respectively) derived from iPS cells of healthy subject and DM1 patient and subjected to staining with anti-αA crystallin antibody and counterstaining of nucleus with DAPI. The scale bar in Fig. 9B is 200 µm. From Fig. 9B, it was confirmed that the flat cells observed in Fig. 9A are lens epithelial cells and lens fiber cells because they express αA-crystallin.

Fig. 9C shows fluorescence microscope photographs of lens epithelial cells and lens fiber cells ⁻(HC and DM1, respectively) derived from iPS cells of healthy subject and DM1 patient that underwent FISH and DAPI counterstaining. In HC, RNA aggregate was not detected, but RNA aggregate was observed in DM1.

### Example 8 Evaluation of ASO using iPS cells derived from DM1 patient (4) lens epithelial cells and lens fiber cells

### (8.1) material and method

At 48 hr after administration of 10 nM ASO to the lens epithelial cells and lens fiber cells derived from iPS cells of the DM1 patient described in Example 7 by lipofection, FISH using Cy3 labeled (CAG)₆-CA DNA/LNA probe was performed in the same manner as in Example 7.

### (8.2) results

As shown in Fig. 9C, RNA aggregates were detected in the lens epithelial cell and lens fiber cells (DM1) derived from the iPS cells of DM1 patient, but RNA aggregates almost disappeared when treated with ASO-4M.

Fig. 9D, left, is a bar graph showing the mean±standard deviation of RNA aggregates per cell nucleus calculated from the FISH results of each of HC, DM1, and DM1+ASO-4M in Fig. 9C. The ASO-4M treatment remarkably decreased the average number of RNA aggregates per cell nucleus in the lens epithelial and fiber cells derived from the cells of DM1 patient. Fig. 9D, right, is a bar graph showing the mean±standard deviation of the percentage of RNA aggregates in all cells, calculated from the FISH results of each of HC, DM1, and DM1+ASO-4M in Fig. 9C. The ASO-4M treatment remarkably decreased the percentage of the number of cells in which RNA aggregates were detected with respect to the total number of lens epithelial and fiber cells derived from the cells of DM1 patient. From the above results, it was concluded that ASO-4M treatment can also decrease RNA aggregates in the lens epithelial and fiber cells derived from the cells of DM1 patient.

### Example 9 Differentiation of skin organoid from iPS cells derived from myotonic dystrophy patient

### (9.1) material and method

Skin organoids were generated based on Lee, J. et al. (Nature, 582: 399-404. (2020)). iPS cells derived from DM1 patient and healthy subject were dissociated using Accutase, suspended in Essential 8 Flex (Gibco) medium supplemented with 20 µM Y27632 and 100 µg/ml Normocin (hereinafter to be referred to as E8-20Y medium), 3,500 viable cells, as confirmed by trypan blue staining, in 100 µL of E8-20Y medium per well were seeded in a round-bottom 96-well plate, and aggregate formation was promoted by centrifugation at 100 rcf of the plate. After 24 hr, 100 µL of Essential 8 Flex (Gibco) medium supplemented with 100 µg/ml of Normocin (hereinafter to be referred to as E8Y medium) was added and further cultured. After 24 hr, the cell aggregates were individually collected and transferred to a new round-bottom 96-well plate together with 100 µL of Essential 6 (Gibco) medium supplemented with 2% Matrigel, 10 µM SB (Stemgent), 4 ng/mL b-FGF, and 2.5 ng/mL BMP-4 (hereinafter to be referred to as E6 medium) per well to initiate differentiation into skin organoids. In order to induce cranial neural crest cell formation on day 3 of differentiation, E6 medium supplemented with 200 ng/ml of LDN (BMP inhibitor, Stemgent) and 50 µg/ml of FGF was added at 25 µL per well to 125 µL finally. On day 6 of differentiation, 75 µL of fresh E6 medium was added to make the final volume 200 µL. Half of the medium was changed on days 8 and 10. On day 12, all aggregates were transferred to individual wells of a 24-well low adhesive plate in 500 µl of organoid maturation medium (OMM) containing 1% Matrigel to induce epidermal self-assembly. To culture the aggregates in suspension and maintain constant medium circulation, the 24-well plates were placed on an orbital shaker with shaking at a speed of 65 rpm in a 37°C incubator containing 5% CO2. OMM is a medium obtained by adding 1X GlutaMax^{™} (Gibco), 0.5X B-27 Minus Vitamin A (Gibco), 0.5X N2 (Gibco) supplements, 0.1 mM 2-Mercaptoethanol (Gibco), and 100 µg/mL Normocin to a mixed medium of Advanced DMEM/F12 (Gibco) and Neurobasal (Gibco) at a ratio of 1:1. On day 15 of differentiation, half of the spent medium was supplemented with OMM containing 1% Matrigel. After day 18, half the medium was replaced with fresh OMM medium without Matrigel every 3 days (from day 18 to day 45) or every other day (from day 45 to day 150 or thereafter), and all the medium was replaced once a week from day 45. As aggregates matured and grew larger, it was sometimes necessary to increase the total volume of medium per well to 1 mL after day 80.

The differentiation of skin organoids was confirmed by the formation of hair follicle structure. In addition, frozen sections of skin organoid were analyzed in the same manner as in Example 5 by using the detection method of nuclear RNA aggregates.

### (9.2) results

As shown in Fig. 10A, hair follicle structures were formed in skin organoids derived from iPS cells of healthy subject and DM1 patient (HC and DM1, respectively) after 4 months-of culture under differentiation conditions. As shown in Figs. 10B and 10C, RNA aggregates were detected in sections of skin organoids derived from iPS cells of DM1 patient after 4 months of culture, but RNA aggregates were not detected in sections of skin organoids derived from skin iPS cells of healthy subject.

### Example 10 Evaluation of ASO using iPS cells derived from DM1 patient (5) skin organoid

### (10.1) material and method

Differentiation of skin organoids from iPS cells derived from healthy subject and myotonic dystrophy patient, and FISH using Cy3 labeled (CAG)₆-CA DNA/LNA probe to detect nuclear RNA aggregates were performed in the same manner as in Example 9. For ASO treatment of organoids, organoids were spin-cultured in an NB medium containing 500 nM ASO-4M.

### (10.2) results

RNA aggregates decreased in ASO-4M-treated skin organoids (DM1+ASO-4M) in Fig. 10B, left. Fig. 10C is a bar graph showing the mean±standard deviation of the percentage of cells in which RNA aggregates are detected in all cells, calculated from the FISH results of each of HC, DM1, and DM1+ASO-4M in Fig. 10B. From Fig. 10C, it was confirmed that the formation of RNA aggregates in the skin organoids derived from the iPS cells of DM1 patient significantly decreased by ASO-4M treatment.

### Example 11 Differentiation of neuron from iPS cells derived from myotonic dystrophy patient

### (11.1) material and method

Neurons differentiated from the iPS cells derived from DM1 patient and healthy subject were generated based on Imamura, K. et al.(Sci Rep., 6:34904. (2016)). Fig. 11A is a conceptual diagram showing procedures for an experimental system for differentiation of undifferentiated cells of iPS cells (DM-1 to -3) derived from DM1 patient and iPS cells (HC-1 to -3) derived from healthy subject into nerve cells, using a tetracycline-inducible NGN2 expression system. As shown in Fig. 11A, "B27/N2/NB+DOX" and "B27/NB+DOX" show the periods of culture in a medium for nerve cell induction supplemented with doxycycline as a differentiation-inducing factor, and "B27/NB" shows the periods of culture in a medium for differentiated nerve cells. Tetracycline-inducible NGN2expression system was introduced into undifferentiated iPS cells by transfection, the medium was changed to a medium for nerve cell induction, differentiation induction was initiated on day 0, the differentiation induction was terminated on day 8 by changing to a medium for nerve cells without coritaining a differentiation-inducing factor, and analysis was performed on day 11.

### (11.2) results

Fig. 11B is a fluorescence microscope photograph of neuron differentiated from iPS cells derived from DM1 patient that underwent staining with an anti-tubulin βIII antibody and DAPI counterstaining. The scale bar is 100 um. As shown in Fig. 11B, according to the procedure of this Example, neurons expressing tubulin βIII and having elongated nerve axon are homogeneously obtained. Fig. 11C shows synthetic images of fluorescence microscope photographs of neurons differentiated from iPS cells (HC-1 to -3) derived from healthy subject and iPS cells (DM-1 to -3) derived from DM1 patient that underwent staining with anti-tubulin βIII antibody (upper row), anti-MAP2 antibody (middle row), or anti-tubulin βIII antibody and anti-Tbr1 antibody, and DAPI counterstaining. As shown in Fig. 11C, iPS cells derived from DM1 patient undergo differentiation into neurons with not much difference from iPS cells derived from healthy subject.

Fig. 11D shows fluorescence microscope photographs of neurons differentiated from iPS cells (HC-1 to -3) derived from healthy subject and iPS cells (DM-1 to -3) derived from DM1 patient that underwent FISH using Cy3 labeled (CAG)₆-CA DNA/LNA probe and DAPI counterstaining. From Fig. 11D, similar to Examples 2 and 3, in the neurons derived from iPS cells DM-2 from among the iPS cells derived from DM1 patients, both the average number of RNA aggregates per cell nucleus and the percentage of RNA aggregates in total cells are markedly smaller than in the neurons derived from iPS cells DM-1 and DM-3. Unlike Examples 2 and 3, there was little difference in the average number of RNA aggregates per cell nucleus and the percentage of RNA aggregates in total cells, in the neurons derived from iPS cells DM-1 and DM-3.

### Example 12 Evaluation of ASO using iPS cells derived from DM1 patient (5) neuron

### (12.1) material and method

On day 8 after differentiation induction into neuron derived from iPS cells of DM1 patient described in Example 11, 10 nM ASO was administered by lipofection, and FISH using Cy3 labeled (CAG)₆-CA DNA/LNA probe was performed on day 11 in the same manner as in Example 7.

### (12.2) results

Fig. 11F, upper left, shows a synthetic image (Control) of fluorescence microscope photograph of iPS cells (DM-1) derived from DM1 patient and treated with Control ASO that underwent FISH using Cy3 labeled (CAG)₆-CA DNA/LNA probe, staining with anti-tubulin βIII antibody, and DAPI counterstaining. Fig. 11F, upper right, shows a synthetic image (ASO-4M) of fluorescence microscope photograph of iPS cells (DM-1) derived from DM1 patient and treated with ASO-4M (same as ASO-4) that underwent FISH using Cy3 labeled (CAG)₆-CA DNA/LNA probe, staining with anti-tubulin βIII antibody, and DAPI counterstaining. As shown in Fig. 11F, RNA aggregates remarkably decreased in neurons by ASO-4M treatment.

### [Industrial Applicability]

As shown in the foregoing Examples of the present specification, the ASO treatment of the present invention could reduce nuclear RNA aggregates in undifferentiated iPS cells derived from DM1 patients, and skeletal muscle cells, lens epithelial cells, lens fiber cells, neurons, neuromuscular organoids, and skin organoids, each differentiated from the iPS cells. In addition, the ASO treatment could recover the satellite cells that had decreased in the neuromuscular organoids derived from DM1 patients to the same level as in the neuromuscular organoids derived from healthy subjects. Therefore, the pharmaceutical composition of the present invention is useful for treating diseases including myotonic dystrophy type 1 disease. Furthermore, the iPS cells of the present invention or the cells and organoids differentiated from the cells are useful as an ASO assay system for treatment in not only myotonic dystrophy type 1 disease, but also all other triplet diseases for which ASO treatment is considered to be effective.

This application is based on a patent application No. 2020-213000 filed in Japan on December 22, 2020, the contents of which are incorporated in full herein by reference.

## Claims

1. A pharmaceutical composition comprising an oligonucleotide having any base sequence of a repeated sequence in which cytosine-adenine-guanine trinucleotides are repeated 5 to 13 times, from the 5' end to the 3' end, and the oligonucleotide has a mixmer structure containing at least two types of RNase H inactive nucleotide analogs.

2. The pharmaceutical composition according to claim 1, wherein the oligonucleotide is
(1) an oligonucleotide consisting of a base sequence of any of SEQ ID NOs: 1 to 9,
(2) an oligonucleotide consisting of a base sequence consisting of adenine-guanine, a base sequence of any of SEQ ID NOs: 1 to 9, and cytosine, from the 5' end to the 3' end, or
(3) an oligonucleotide consisting of a base sequence consisting of guanine, a base sequence of any of SEQ ID NOs: 1 to 9, and cytosine-adenine, from the 5' end to the 3' end.

3. The pharmaceutical composition according to claim 1 or 2, wherein the RNase H inactive nucleotide analog is a nucleotide analog modified at the 2'-position of ribose and/or a modified nucleotide analog bridged between the 2'-position and the 4'-position of ribose.

4. The pharmaceutical composition according to claim 3, wherein the nucleotide analog with a modified oxygen atom at the 2'-position of ribose is a 2'-OMe-nucleotide analog and/or a MOE-nucleotide analog.

5. The pharmaceutical composition according to claim 3, wherein the modified nucleotide analog bridged between the 2'-position and the 4'-position of ribose is β-D-oxy-L-LNA, β-D-ENA and/or R type cEt.

6. The pharmaceutical composition according to any one of claims 3 to 5, wherein the nucleotide analogs modified at the 2'-position of ribose all have the same modified sugar.

7. The pharmaceutical composition according to any one of claims 3 to 5, wherein the nucleotide analogs modified at the 2'-position of ribose have at least two different modified sugars.

8. The pharmaceutical composition according to any one of claims 3 to 7, wherein the modified nucleotide analogs bridged between the 2'-position and the 4'-position of ribose all have the same modified sugar.

9. The pharmaceutical composition according to any one of claims 3 to 7, wherein the modified nucleotide analogs bridged between the 2'-position and the 4'-position of ribose have at least two different modified sugars.

10. The pharmaceutical composition according to any one of claims 3 to 9, wherein the oligonucleotide comprises β-D-ENA-cytidine, 2'-OMe-adenosine, and 2'-OMe-guanosine.

11. The pharmaceutical composition according to claim 10, wherein the cytidines in the oligonucleotide are all β-D-ENA-cytidines, adenosines are all 2'-OMe-adenosines, and guanosines are all 2'-OMe-guanosines.

12. The pharmaceutical composition according to any one of claims 1 to 11, wherein the oligonucleotide comprises at least one type of nucleotide bond selected from the group consisting of phosphorothioate, phosphorodithioate, and boranophosphate.

13. The pharmaceutical composition according to any one of claims 1 to 12, comprising a pharmaceutically acceptable carrier or diluent.

14. The pharmaceutical composition according to claim 13, wherein the carrier comprises a cellular membrane permeation carrier and/or a carrier for intranuclear delivery.

15. The pharmaceutical composition according to any one of claims 1 to 14, wherein the pharmaceutical composition is delivered by parenteral administration selected from the group consisting of transdermal administration, intraocular administration, intra-lens administration, gastrointestinal tract intramucosal administration, gastrointestinal tract submucosal administration, subcutaneous administration, intravenous administration, intraarterial administration, intramuscular administration, intraperitoneal administration, intracranial administration, and intrathecal administration.

16. The pharmaceutical composition according to any one of claims 1 to 15, wherein the pharmaceutical composition is used for treating any of the symptoms of myotonic dystrophy type 1 disease.
